# EUROPÄISCHE PATENTSCHRIFT

(11) **EP 1 888 514 B1**
(45) Veröffentlichungstag und Bekanntmachung des Hinweises auf die Patenterteilung: **18.03.2009**
(21) Anmeldenummer: 06753677.1
(22) Anmeldetag: 17.05.2006
(51) Int. Cl.: C07C 275/60, A61K 31/17, A61P 29/00

(54) **SALZE SUBSTITUIERTER ALLOPHANSÄUREESTER UND DEREN VERWENDUNG IN ARZNEIMITTELN**
SALTS OF SUBSTITUTED ALLOPHANATES AND THEIR USE IN DRUGS
SELS D'ESTERS D'ACIDE ALLOPHANIQUE SUBSTITUES, ET LEUR UTILISATION POUR PRODUIRE DES MEDICAMENTS

(30) Priorität: 18.05.2005 DE 102005023588
(43) Veröffentlichungstag der Anmeldung: 20.02.2008
(73) Patentinhaber: Grünenthal GmbH, 52078 Aachen (DE)
(72) Erfinder: FRANK, Robert, 52070 Aachen (DE); JOSTOCK, Ruth, 52223 Stolberg (DE); SCHICK, Hans, 13086 Berlin (DE); THEIL, Fritz, 12247 Berlin (DE); GRÖGER, Olga, 12587 Berlin (DE); KUDICK, Rene, 15537 Grünhelde (DE); SONNENSCHEIN, Helmut, 10245 Berlin (DE); HENKEL, Birgitta, 12555 Berlin (DE)
(74) Vertreter: Bülle, Jan
(86) Internationale Anmeldenummer: PCT/EP2006/004657
(87) Internationale Veröffentlichungsnummer: WO 2006/122772

(56) Entgegenhaltungen:
- DE-A1- 10 131 462
- US-A- 4 684 728
- US-A- 5 494 922

## Beschreibung

Die vorliegende Erfindung betrifft Salze substituierter Allophansäureester, Verfahren zu ihrer Herstellung, Arzneimittel enthaltend diese Verbindungen sowie die Verwendung dieser Verbindung zur Herstellung von Arzneimitteln.

Die Behandlung von Schmerz, insbesondere von neuropathischem Schmerz, hat In der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception In letzter Zeit erschienen sind.

US 4,684,728 offenbart Allophansäureester, welche u.a. in Arzneimitteln verwendet werden können. US 5,494,922 betrifft Allophansäureester, die als Inhibitoren der Anbindung von Fibrinogen an Glykoprotein IIb/IIIa fungieren. DE 101 31 462 A1 betrifft Phenolderivate, welche als Arzneimittel, beispielsweise zur Behandlung von Depression, verwendet werden können.

Einen geeigneten Ansatzpunkt zur Behandlung von Schmerz; insbesondere von Schmerz ausgewählt aus der Gruppe bestehend aus akutem Schmerz, chronischem Schmerz, neuropathischem Schmerz und visceralem Schmerz, besonders bevorzugt von neuropathischem Schmerz; stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanlllolde wie z.B. Capsaicin, Hitze und Protonen stimuliert und spielt eine zentrale Rolle bei der Schmerzentstehung. Darüber hinaus Ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Haminkontinenz.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass sich Salze substituierter Allophansäureester der nachstehend angegebenen Zusammensetzung zur Bekämpfung von Schmerzen eignen und eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden.

Ein Gegenstand der vorliegenden Erfindung sind daher Salze substituierter Allophansäureester aus einem kationischen Salzpartner der allgemeinen Formel I, 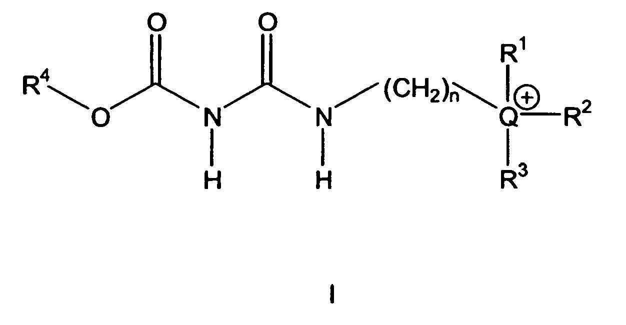 worin
n = 1, 2, 3, 4, 5 oder 6 ist;
Q für ein Stickstoffatom oder ein Phosphoratom steht;
- R¹, R², R³,: unabhängig voneinander, jeweils für einen linearen oder verzweigten C₁₋₅-Alkyl-Rest stehen;
oder zwei dieser Reste zusammen mit dem sie verbindenden Atom Q als Ringglied einen 4-, 5-, 6- oder 7-gliedrigen, gesättigten oder ungesättigten, unsubstituierten oder einfach oder mehrfach substituierten, ggf. wenigstens ein weiteres Heteroatom als Ringglied aufweisenden Heterozyklus bilden und jeweils der verbleibende Rest die vorstehend genannte Bedeutung hat;
- R⁴: für einen linearen oder verzweigten, gesättigten oder ungesättigten, unsubstituierten oder einfach oder mehrfach substituierten, ggf. wenigstens ein Heteroatom als Kettenglied aufweisenden aliphatischen Rest;
für einen ungesättigten oder gesättigten, unsubstituierten oder einfach oder mehrfach substituierten, ggf. wenigstens ein Heteroatom als Ringglied aufweisenden cycloaliphatischen Rest, der über eine lineare oder verzweigte Alkylen-Gruppe gebunden sein kann,
für einen unsubstituierten oder wenigstens einfach oder mehrfach substituierten, ggf. über eine lineare oder verzweigte Alkylen-Gruppe gebundenen Aryl-Rest oder Heteroaryl-Rest steht, oder
für eine Gruppe -(CH₂)_{q}-Aᵣ-(CH₂)ₛ-B-(CH₂)ᵣCᵤ-R⁵ steht, in der
q, s und t, unabhängig voneinander, jeweils für 0, 1, 2, 3, 4, 5 oder 6 stehen;
r und u, unabhängig voneinander, jeweils für 0 oder 1 stehen;
A und C, unabhängig voneinander, jeweils für O, S oder NH stehen;
B für eine lineare oder verzweigte, unsubstituierte oder einfach oder mehrfach substituierte Alkylen-, Alkenylen- oder Alkinylen-Gruppe steht; für einen ungesättigten oder gesättigten, unsubstituierten oder einfach oder mehrfach substituierten, ggf. wenigstens ein Heteroatom als Ringglied aufweisenden cycloaliphatischen Rest; oder für einen unsubstituierten oder einfach oder mehrfach substituierten Aryl-Rest oder Heteroaryl-Rest steht; und
R⁵ für einen linearen oder verzweigten, gesättigten oder ungesättigten, unsubstituierten oder einfach oder mehrfach substituierten aliphatischen Rest steht,
jeweils ggf. in Form eines seiner reinen Stereoisomeren, insbesondere Enantiomeren oder Diastereomeren, seines Racemates oder in Form einer Mischung von Stereoisomeren, insbesondere der Enantiomeren und/oder Diastereomeren, in einem beliebigen Mischungsverhältnis,
und einem anionischen Salzpartner.

Sofern einer oder beide der Substituenten R⁴ und R⁵ für einen gesättigten oder ungesättigten aliphatischen Rest, d.h. für einen Alkyl-,Alkenyl- oder Alkinyl-Rest, stehen, kann dieser bevorzugt mit ggf. 1, 2, 3, 4, 5, 6, 7, 8 oder 9 Substituenten unabhängig voneinander ausgewählt aus der Gruppe bestehend aus F, Cl, Br, I,-CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein. Alkenyl-Reste weisen wenigstens eine, beispielsweise 1, 2, 3 oder 4, C-C-Doppelbindungen und Alkinyl-Reste weisen wenigstens eine, beispielsweise 1, 2, 3 oder 4 C-C-Dreifachbindungen auf.

Sofern der Substituent R⁴ für einen aliphatischen Rest steht, der wenigstens 1, beispielsweise 1, 2, 3 oder 4 Heteroatome als Kettenglied(er) aufweist, können diese unabhängig voneinander bevorzugt ausgewählt werden aus der Gruppe bestehend aus Sauerstoff, Schwefel und Stickstoff.

Sofern zwei der Reste R¹, R² und R³ zusammen mit dem sie verbindenden Atom Q als Ringglied einen 4-, 5-, 6- oder 7-gliedrigen, gesättigten oder ungesättigten, Heterozyklus bilden, der einfach oder mehrfach, beispielsweise 1-, 2-, 3-, 4- oder 5-fach substituiert ist, können dessen Substituenten unabhängig voneinander ausgewählt werden aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -SCF₃,-SH, -S-CH₃, -S-C₂H₅, -S-C(CH₃)₃, Methyl, Ethyl, n-Propyl, Isopropyl, n-Butyl, sec-Butyl, Isobutyl, tert-Butyl, n-Pentyl, sec-Pentyl, -C(=O)-OH, -C(=O)-O-CH₃, -C(=O)-O-C₂H₅, -N(CH₃)₂, -N(C₂H₅)₂, -N(H)(CH₃), -N(H)(C₂H₅), -O-Phenyl, -O-Benzyl, Phenyl und Benzyl, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, Isobutyl, tert-Butyl,-O-CH₃, -O-C₂H₅, -O-C(CH₃)₃, -O-CF₃, -S-CF₃, Phenyl und -O-Benzyl substituiert sein kann. Sofern der mit Q gebildete Heterozyklus eines oder mehrere, beispielsweise 1 oder 2, weitere Heteroatome als Ringglieder aufweist, können diese unabhängig voneinander bevorzugt ausgewählt werden aus der Gruppe bestehend aus Stickstoff, Sauerstoff und Schwefel.

Sofern einer oder beide der Reste R⁴ und B für einen ungesättigten oder gesättigten, ggf. wenigstens ein Heteroatom als Ringglied aufweisenden cycloaliphatischen Rest stehen oder einen solchen aufweisen, der einfach oder mehrfach, beispielsweise 1-, 2-, 3-, 4- oder 5-fach substituiert ist, können dessen Substituenten unabhängig voneinander ausgewählt werden aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂,-O-CF₃, -SCF₃, -SH, -S-CH₃, -S-C₂H₅, -S-C(CH₃)₃, Methyl, Ethyl, n-Propyl, Isopropyl, n-Butyl, sec-Butyl, Isobutyl, tert-Butyl, n-Pentyl, sec-Pentyl, -C(=O)-OH, -C(=O)-O-CH₃, -C(=O)-O-C₂H₅, -N(CH₃)₂, -N(C₂H₅)₂, -N(H)(CH₃), -N(H)(C₂H₅), -O-Phenyl, -O-Benzyl, Phenyl und Benzyl, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅,-CN, -NO₂, Methyl, Ethyl, n-Propyl, Isopropyl, n-Butyl, Isobutyl, tert-Butyl,-O-CH₃, -O-C₂H₅, -O-C(CH₃)₃, -O-CF₃, -S-CF₃, Phenyl und -O-Benzyl substituiert sein kann. Sofern dieser cycloaliphatische Rest eines oder mehrere, beispielsweise 1 oder 2, Heteroatome als Ringglieder aufweist, können diese unabhängig voneinander bevorzugt ausgewählt werden aus der Gruppe bestehend aus Stickstoff, Sauerstoff und Schwefel.

Beispielhaft seien die Reste Cyclopropyl, Cylobutyl, Cyclopentyl, Cyclohexyl, Cyclohexyl, Cyclooctyl, Cyclononyl, Cyclopentenyl, Cyclohexenyl, Cycloheptenyl, Cyclooctenyl, Imidazolinyl, Aziridinyl, Azetidinyl, Pyrrolidinyl, Piperidinyl, Azepanyl, Azokanyl, Piperazinyl, Tetrahydrofuranyl (Tetrahydrofuryl), Tetrahydrothienyl (Tetrahydrothiophenyl), Morpholinyl und Thiomorpholinyl genannt.

Sofern einer oder beide der Reste R⁴ und B für einen Aryl- oder Heteroaryl-Rest stehen oder einen solchen aufweisen, der einfach oder mehrfach, beispielsweise 1-, 2-, 3-, 4- oder 5-fach substituiert ist, können dessen Substituenten unabhängig voneinander ausgewählt werden aus der Gruppe bestehend aus F, Cl, Br, I, -CN,-CF₃, -SF₅, -OH, -O-CH₃, -O-C₂H₅, -O-C(CH₃)₃, -NH₂, -NO₂, -O-CF₃, -SCF₃, -SH, -S-CH₃, -S-C₂H₅, -S-C(CH₃)₃, Methyl, Ethyl, n-Propyl, Isopropyl, n-Butyl, sec-Butyl, Isobutyl, tert-Butyl, n-Pentyl, sec-Pentyl, -C(=O)-OH, -C(=O)-O-CH₃, -C(=O)-O-C₂H₅, -N(CH₃)₂, -N(C₂H₅)₂, -N(H)(CH₃), -N(H)(C₂H₅), -O-Phenyl, -O-Benzyl, Phenyl und Benzyl, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, Isobutyl, tert-Butyl,-O-CH₃, -O-C₂H₅, -O-C(CH₃)₃, -O-CF₃,-S-CF₃, Phenyl und -O-Benzyl substituiert sein kann. Der Heteroaryl-Rest kann eines oder mehrere, beispielsweise 1, 2 oder 3, Heteroatome als Ringglieder aufweisen, die unabhängig voneinander bevorzugt ausgewählt werden aus der Gruppe bestehend aus Stickstoff, Sauerstoff und Schwefel.

Beispielhaft seien als Aryl-Reste Phenyl, 1-Naphthyl und 2-Naphthyl genannt.

Als Heteroaryl-Reste seien beispielhaft Pyrrolyl, Indolyl, Furyl (Furanyl), Benzo[b]furanyl, Thienyl (Thiophenyl), Benzo[b]thienyl, Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl und Chinazolinyl genannt.

Substituierte Alkylen-, Alkenylen- oder Alkinylen-Gruppen können beispielsweise mit 1, 2 oder 3 Substituenten unabhängig voneinander ausgewählt aus der Gruppe bestehend aus F, Cl, Br, OH, -O-C₁₋₃-Alkyl, SH und -S-C₁₋₃-Alkyl substituiert.

Bevorzugt sind erfindungsgemäße Salze auf Basis von kationischen Salzpartnern der oben angegebenen allgemeinen Formel I, in denen
- R¹, R², R³,: unabhängig voneinander, jeweils für einen Alkyl-Rest stehen, der ausgewählt ist aus der Gruppe bestehend aus Methyl, Ethyl, n-Propyl und iso-Propyl,
oder zwei dieser Reste zusammen mit dem sie verbindenden Atom Q als Ringglied einen 4-, 5-, 6- oder 7-gliedrigen, gesättigten oder ungesättigten, unsubstituierten oder einfach oder mehrfach substituierten, ggf. wenigstens ein weiteres Heteroatom als Ringglied aufweisenden Heterozyklus bilden und jeweils der verbleibende Rest für einen Alkyl-Rest steht, der ausgewählt ist aus der Gruppe bestehend aus Methyl, Ethyl, n-Propyl und iso-Propyl,
und jeweils Q, n, R⁴, R⁵, q, r, s, t, u, A, B und C die vorstehend genannte Bedeutung haben.

Bevorzugt sind ferner erfindungsgemäße Salze auf Basis von kationischen Salzpartnern der oben angegebenen allgemeinen Formel I, in denen
- R⁴: für einen linearen oder verzweigten, gesättigten oder ungesättigten, unsubstituierten oder einfach oder mehrfach substituierten aliphatischen C₁₋₃₀ Rest;
für einen ungesättigten oder gesättigten, unsubstituierten oder einfach oder mehrfach substituierten, ggf. ein, zwei oder drei Heteroatome als Ringglied aufweisenden, 5-, 6- oder 7-gliedrigen cycloaliphatischen Rest, der über eine lineare oder verzweigte C₁₋₃ Alkylen-Gruppe gebunden sein kann,
für einen unsubstituierten oder wenigstens einfach substituierten, ggf. über eine lineare oder verzweigte C₁₋₃-Alkylen-Gruppe gebundenen 5- oder 6-gliedrigen Aryl-Rest oder Heteroaryl-Rest steht, oder
für eine Gruppe -(CH₂)_{q}-Aᵣ-(CH₂)ₛ-B-(CH₂)ₜ-Cᵤ-R⁵ steht, in der
q, s und t, unabhängig voneinander, jeweils für 0, 1, 2, 3, 4, 5 oder 6 stehen;
r und u, unabhängig voneinander, jeweils für 0 oder 1 stehen;
A und C, unabhängig voneinander, jeweils für O oder S stehen;
B für eine lineare oder verzweigte C₁₋₆-Alkylen-, C₂₋₆-Alkenylen- oder C₂₋₆-Alkinylen-Gruppe steht; die mit 1, 2 oder 3 Substituenten unabhängig voneinander ausgewählt aus der Gruppe bestehend aus F, Cl, Br, OH, -O-C₁₋₃-Alkyl, SH und -S-C₁₋₃-Alkyl substituiert sein kann,
für einen (hetero)cycloaliphatischen Rest ausgewählt aus der Gruppe bestehend aus Cyclopropyl, Cylobutyl, Cyclopentyl, Cyclohexyl, Cyclohexyl, Cyclooctyl, Cyclononyl, Cyclopentenyl, Cyclohexenyl, Cycloheptenyl, Cyclooctenyl, Imidazolinyl, Aziridinyl, Azetidinyl, Pyrrolidinyl, Piperidinyl, Azepanyl, Azokanyl, Piperazinyl, Tetrahydrofuranyl (Tetrahydrofuryl), Tetrahydrothienyl (Tetrahydrothiophenyl), Morpholinyl und Thiomorpholinyl,
oder für einen (Hetero)aryl-Rest ausgewählt aus der Gruppe bestehend aus Pyrrolyl, Indolyl, Furyl (Furanyl), Benzo[b]furanyl, Thienyl (Thiophenyl), Benzo[b]thienyl, Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl und Chinazolinyl steht, und
R⁵ für einen linearen oder verzweigten, gesättigten oder ungesättigten, unsubstituierten oder einfach oder mehrfach substituierten aliphatischen C₁₋₃₀-Rest steht, und
und jeweils R¹, R², R³, Q, n, q, r, s, t, u, A, B und C die vorstehend genannte Bedeutung haben.

Weiterhin bevorzugt sind ferner erfindungsgemäße Salze auf Basis von kationischen Salzpartnern der oben angegebenen allgemeinen Formel I, in denen
n = 1, 2, 3, 4, 5 oder 6 ist;
Q für N oder P steht;
- R¹, R², R³,: unabhängig voneinander, jeweils für einen Alkyl-Rest stehen, der ausgewählt ist aus der Gruppe bestehend aus Methyl, Ethyl, n-Propyl und iso-Propyl,
oder zwei dieser Reste zusammen mit dem sie verbindenden Atom Q als Ringglied einen 4-, 5-, 6- oder 7-gliedrigen, gesättigten, unsubstituierten Heterozyklus bilden und jeweils der verbleibende Rest für einen Alkyl-Rest steht, der ausgewählt ist aus der Gruppe bestehend aus Methyl, Ethyl, n-Propyl und iso-Propyl,
- R⁴: für einen linearen oder verzweigten, gesättigten oder ungesättigten aliphatischen C₁₋₂₀ Rest;
für einen ggf. über eine C₁₋₃-Alkylen-Gruppe gebundenen Aryl- oder Heteroaryl-Rest ausgewählt aus der Gruppe bestehend aus Phenyl, Naphthyl, Furanyl, Thiophenyl und Pyridinyl, wobei der zyklische Teil dieser Reste jeweils unsubstituiert oder mit 1, 2, 3, 4 oder 5 Substituenten ausgewählt aus der Gruppe bestehend aus F, Cl, Br, linearem oder verzweigtem C₁₋₃-Alkyl, linearem oder verzweigtem C₁₋₃-Alkoxy, Phenyl, Phenoxy, Benzyl und Benzyloxy substituiert sein kann;
für eine Gruppe -(CH₂)_{q}-Aᵣ-(CH₂)ₛ-B-(CH₂)ₜ-Cᵤ-R⁵ steht, in der
q, s und t, unabhängig voneinander, jeweils für 0, 1 oder 2 stehen;
r und u, unabhängig voneinander, jeweils für 0 oder 1 stehen;
A und C, unabhängig voneinander, jeweils für O stehen;
B für eine lineare oder verzweigte C₁₋₆-Alkylen-, C₂₋₆-Alkenylen- oder C₂₋₆-Alkinylen-Gruppe steht; die mit 1, 2 oder 3 Substituenten unabhängig voneinander ausgewählt aus der Gruppe bestehend aus F, Cl, Br, OH, -O-C₁₋₃-Alkyl, SH und -S-C₁₋₃-Alkyl substituiert sein kann,
für einen (hetero)cycloaliphatischen Rest ausgewählt aus der Gruppe bestehend aus Cyclopropyl, Cylobutyl, Cyclopentyl, Cyclohexyl, Cyclohexyl, Cyclooctyl, Cyclononyl, Cyclopentenyl, Cyclohexenyl, Cycloheptenyl, Cyclooctenyl, Imidazolinyl, Aziridinyl, Azetidinyl, Pyrrolidinyl, Piperidinyl, Azepanyl, Azokanyl, Piperazinyl, Tetrahydrofuranyl (Tetrahydrofuryl), Tetrahydrothienyl (Tetrahydrothiophenyl), Morpholinyl und Thiomorpholinyl,
oder für einen (Hetero)aryl-Rest ausgewählt aus der Gruppe bestehend aus Pyrrolyl, Indolyl, Furyl (Furanyl), Benzo[b]furanyl, Thienyl (Thiophenyl), Benzo[b]thienyl, Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl und Chinazolinyl steht, und
- R⁵: für einen linearen oder verzweigten, gesättigten oder ungesättigten aliphatischen Rest C₁₋₂₀-Rest steht.

Weiterhin bevorzugt sind ferner erfindungsgemäße Salze auf Basis von kationischen Salzpartnern der allgemeinen Formel la 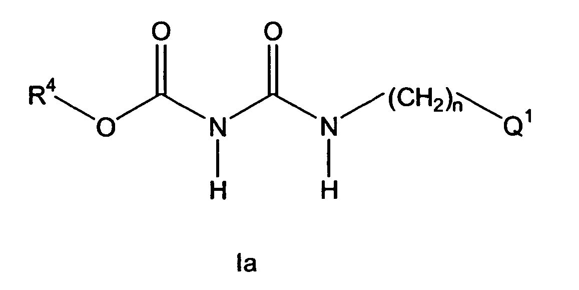 worin
n für 1, 2 oder 3 steht;
Q¹ für einen Rest steht, der ausgewählt ist aus der Gruppe bestehend aus 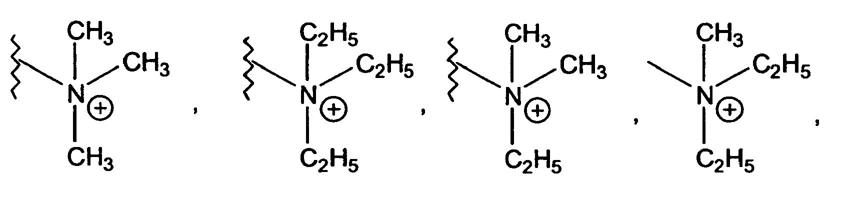 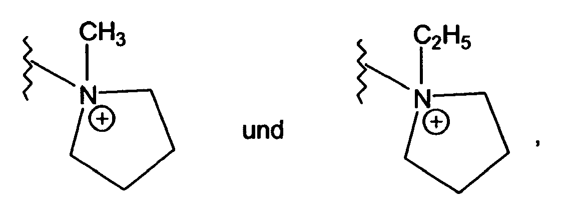 R⁴ für einen linearen oder verzweigten C₁₋₂₀ Alkyl-Rest;
für einen Phenyl- oder Benzyl-Rest, wobei der zyklische Teil dieser Reste unsubstituiert oder mit 1, 2, 3, 4 oder 5 Substituenten ausgewählt aus der Gruppe bestehend aus F, Cl, Br, linearem oder verzweigtem C₁₋₃-Alkyl, linearem oder verzweigtem C₁₋₃-Alkoxy, Phenyl, Phenoxy, Benzyl und Benzyloxy substituiert sein kann;
oder für einen Rest ausgewählt aus der Gruppe bestehend aus 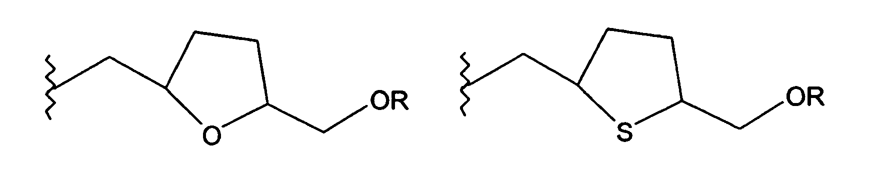 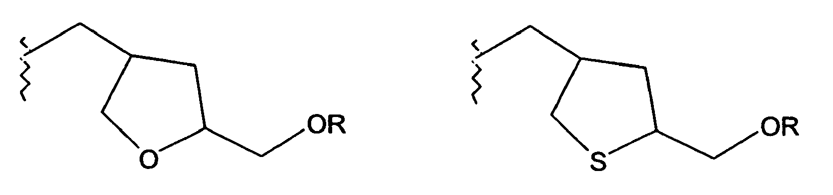 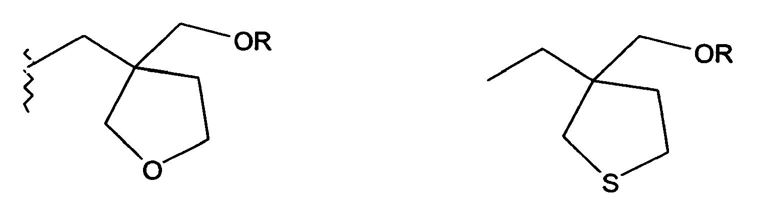 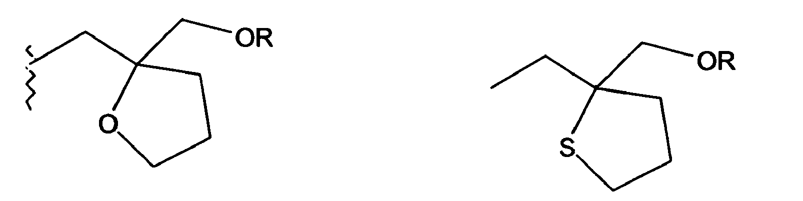 worin R jeweils für einen linearen oder verzweigten C₁₋₂₀-Alkyl-Rest steht.

Weiterhin bevorzugt sind ferner erfindungsgemäße Salze auf Basis von kationischen Salzpartnern der oben angegebenen allgemeinen Formel Ia, in denen
n für 1, 2 oder 3 steht,
Q¹ für einen Rest steht, der ausgewählt ist aus der Gruppe bestehend aus 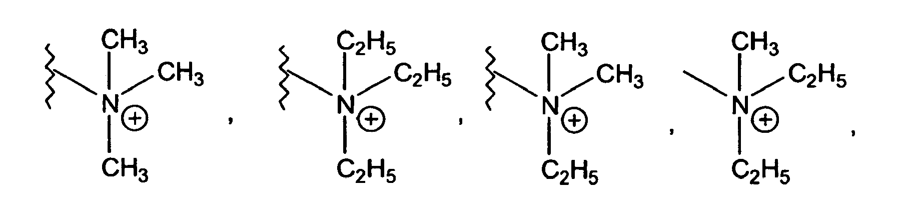 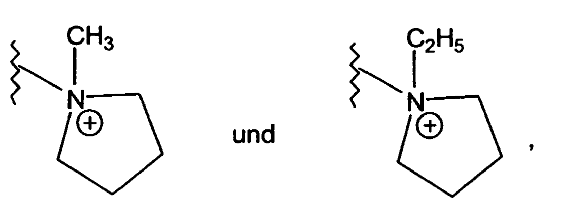 R⁴ für einen Alkyl-Rest ausgewählt aus der Gruppe bestehend aus Methyl, Ethyl, n-Propyl, n-Butyl, n-Pentyl, n-Hexyl, n-Heptyl, n-Octyl, n-Nonyl, n-Decanyl, n-Undecanyl, n-Dodecanyl, n-Tridecanyl, n-Tetradecanyl, n-Pentadecanyl, n-Hexadecanyl, n-Heptadecanyl, n-Octadecanyl, n-Nonadecanyl und n-Eicosanyl;
für einen Phenyl- oder Benzyl-Rest, wobei der zyklische Teil dieser Reste unsubstituiert oder einfach mit einem Substituenten ausgewählt aus der Gruppe bestehend aus F, Cl, Br, linearem oder verzweigtem C₁₋₃-Alkyl, linearem oder verzweigtem C₁₋₃-Alkoxy, Phenyl, Phenoxy, Benzyl und Benzyloxy substituiert sein kann;
oder für einen der folgenden Reste steht, 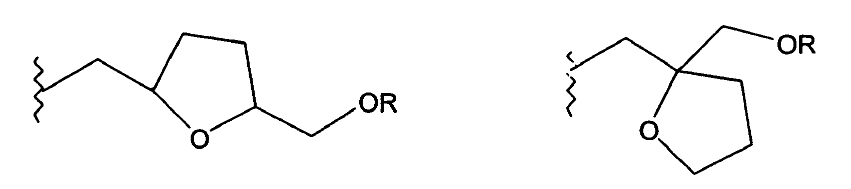 worin R jeweils für einen Alkyl-Rest ausgewählt aus der Gruppe bestehend aus Methyl, Ethyl, n-Propyl, n-Butyl, n-Pentyl, n-Hexyl, n-Heptyl, n-Octyl, n-Nonyl, n-Decanyl, n-Undecanyl, n-Dodecanyl, n-Tridecanyl, n-Tetradecanyl, n-Pentadecanyl, n-Hexadecanyl, n-Heptadecanyl, n-Octadecanyl, n-Nonadecanyl und n-Eicosanyl steht.

Als anionische Salzpartner können übliche, dem Fachmann bekannte, insbesondere pharmazeutisch verträgliche Salzpartner zum Einsatz kommen. Bevorzugt kommt dabei als anionischer Salzpartner ein Halogenid-Ion oder ein Hydroxyl-Ion in Betracht, besonders bevorzugt ein Chlorid-, Bromid- oder Iodid-Ion, ganz besonders bevorzugt ein Iodid-Ion. Ein gegebenener anionischer Salzpartner kann nach üblichen, dem Fachmann bekannten Verfahren auch durch Umsalzung gegen einen anderen anionischen Salzpartner ausgetauscht werden.

Ganz besonders bevorzugt sind Salze substituierter Allophansäureester ausgewählt aus der Gruppe bestehend aus
[1] 4-(Trimethylamino-1-ethyl)allophansäure-dodecylester-iodid,
[2] 4-(Pyrrolidinium-1-ethyl)allophansäure-hexadecylester-iodid,
[3] 4-(Trimethylamino-1-propyl)allophansäure-hexadecylester-iodid,
[4] 4-(Trimethylamino-1-ethyl)allophansäure-octylester-iodid,
[5] 4-(Trimethylamino-1-ethyl)allophansäure-butylester-iodid,
[6] 4-(Trimethylamino-1-ethyl)allophansäure-phenylester-iodid,
[7] 4-Benzyloxycarbonyl-4-(N-methyldimethylammonium-1-ethyl)allophansäure-benzylester-iodid,
[8] 4-(Trimethylamino-1-ethyl)allophansäure-benzylester-iodid,
[9] 4-(Trimethylamino-1-ethyl)allophansäure-(4-phenyl)-phenylester-iodid,
[10] 4-(Trimethylamino-1-ethyl)allophansäure-ethylester-iodid,
[11] 4-(Trimethylamino-1-ethyl)allophansäure-butylester-iodid,
[12] 4-(Trimethylamino-1-ethyl)allophansäure-hexylester-iodid,
[13] 4-(Trimethylamino-1-ethyl)allophansäure-(cis-5-hexadecyloxymethyltetrahydrofuran-2-yl)methylester-iodid,
[14] 4-(Trimethylamino-1-ethyl)allophansäure-3-hexadecyloxy-2-methoxypropan-1-yl-ester-iodid,
[15] 4-(Trimethylamino-1-ethyl)allophansäure-hexadecylester-iodid und
[16] 4-(Trimethylamino-1-ethyl)allophansäure-(2-hexadecyloxymethyltetrahydrofuran-2-yl)methylester-iodid.

Ebenfalls können erfindungsgemäße Salze bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µm aufweisen.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung der erfindungsgemäßen Salze substituierter Allophansäureester, gemäß dem eine Verbindung der allgemeinen Formel II, 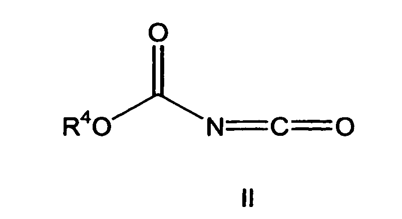 worin R⁴ die vorstehend genannte Bedeutung hat, in einem ggf. absoluten Reaktionsmedium, vorzugsweise in Diethylether, bei niedrigen Temperaturen, vorzugsweise -15 bis + 5 °C; ggf. in Gegenwart einer Base, vorzugsweise einem organischen Amin wie beispielsweise Triethylamin, mit einer Verbindung der allgemeinen Formel III 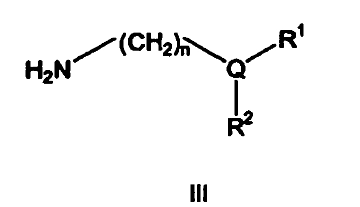 worin n, R¹ und R² die vorstehend genannte Bedeutung haben, zu einer Verbindung der allgemeinen Formel IV, 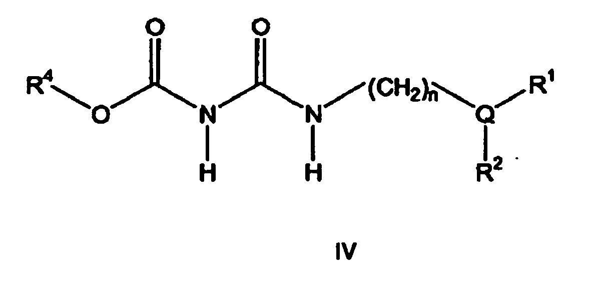 ggf. eines entsprechenden Säureadditionssalzes wie beispielsweise des Hydrochlorids umsetzt, worin R¹, R², R⁴ und n die vorstehend genannte Bedeutung haben, und die so erhaltene Verbindung der allgemeinen Formel IV durch Umsetzung in einem ggf. absolutem Reaktionsmedium wie beispielsweise Dimethylformamid oder Tetrahydrofuran, ggf. in Gegenwart einer Base wie beispielsweise Diisopropylethylamin, mit einem Alkylierungsmittel, vorzugsweise einem C_{1, 2, 3, 4 oder-5}-Alkyliodid, In eine Verbindung der allgemeinen Formel überführt und diese ggf. reinigt und isoliert

Die eingesetzten Chemikalien und Reaktionskomponenten sind käuflich am Markt erhältlich oder können nach üblichen, dem Fachmann bekannten Methoden hergestellt werden. Beispielsweise können die Verbindungen der allgemeinen Formel II gemäß folgendem Schema I erhalten werden:

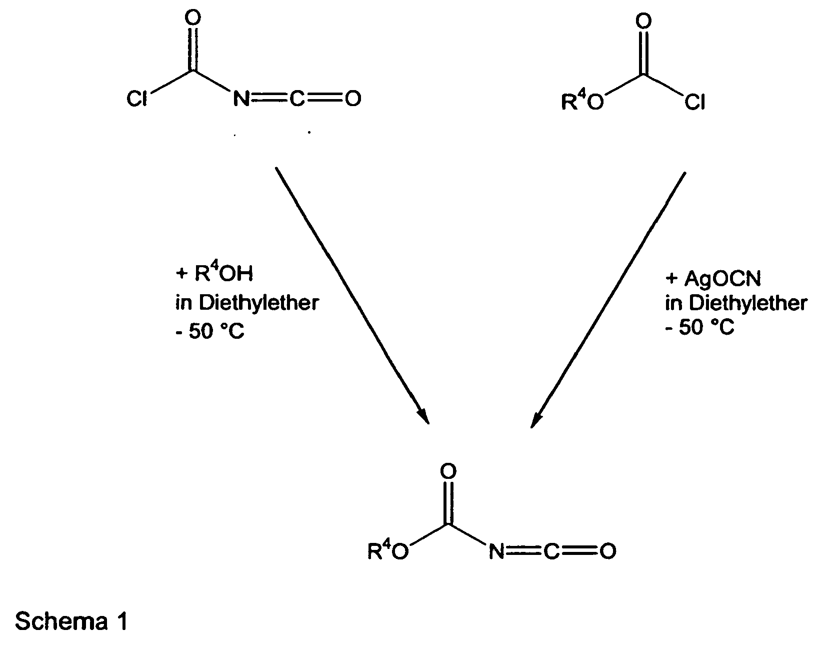

Die vorstehend beschriebenen Umsetzungen können ferner jeweils unter üblichen, dem Fachmann geläufigen Bedingungen, beispielsweise in Hinblick auf Druck, Temperatur, Schutzgasatmosphäre oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können vorteilhafterweise teilweise oder vollständig unter einer Inertgasatmosphäre, vorzugsweise unter Stickstoffatmosphäre oder Argonatmosphäre, durchgeführt werden.

Die erfindungsgemäßen Salze substituierter Allophansäureester sind toxikologisch unbedenklich und eignen sich daher als pharmazeutische Wirkstoffe in Arzneimitteln.

Ein weiterer Gegenstand der vorliegenden Erfindung sind daher Arzneimittel enthaltend wenigstens ein erfindungsgemäßes Salz eines substituierten Allophansäureesters sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1 (VR1/TRPV1)-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens eines erfindungsgemäßen Salzes eines substituierten Allophansäureesters, sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Bevorzugt ist die Verwendung wenigstens eines erfindungsgemäßen Salzes eines substituierten Allophansäureesters, sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens eines erfindungsgemäßen Salzes eines substituierten Allophansäureesters, sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens eines erfindungsgemäßen Salzes eines substituierten Allophansäureesters sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens eines erfindungsgemäßen Salzes eines substituierten Allophansäureesters sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden. Neben wenigstens einem erfindungsgemäßen Salz enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden Salze können in einem Depot, in gelöster Form oder in einem Pflaster, gegebenenfalls unter Zusatz von die Hautpenetration fördernden Mitteln, als geeignete perkutane Applikationszubereitungen vorliegen.

Oral oder perkutan anwendbare Zubereitungsformen können das jeweilige erfindungsgemäße Salz auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remingtons Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge des jeweiligen erfindungsgemäßen Salzes kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in einen kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig abpipettiert und die DRGs werden jeweils mit 500 µL Komplett-Medium versetzt.
Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca ²⁺-Messung im FLIPR-Assay eingesetzt. Die Ca2⁺⁻abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

Ausgehend von der prozentualen Verdrängung durch unterschiedliche Konzentrationen der zu prüfenden Verbindungen der allgemeinen Formel I werden IC₅₀ Hemmkonzentrationen berechnet, die eine 50-prozentige Verdrängung des Capsaicin bewirken.

### II. Funktionelle Untersuchungen am Vanilloid Rezeptor (VR1)

Die agonistische bzw. antagonistische Wirkung der zu untersuchenden Substanzen auf den Vanilloid Rezeptor (VR1) kann mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37°C inkubiert. Anschließend werden die Platten 3 mal mit HBSS-Puffer gewaschen und nach einer weiteren Inkubation von 15 Minuten bei Raumtemperatur zur Ca²⁺⁻-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsazepin berechnet.

### III. Formalin-Test an der Maus

Die Untersuchung zur Bestimmung der antinociceptiven Wirkung der erfindungsgemäßen Salze wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Salze werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen Salze vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lösung) vor Formalinapplikation erhalten.
Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

Im folgenden wird die vorliegende Erfindung anhand von Beispielen erläutert. Diese Erläuterungen sind lediglich beispielhaft und schränken den allgemeinen Erfindungsgedanken nicht ein.

### Beispiele:

### Synthese von Salzen substituierter Allophansäureester:

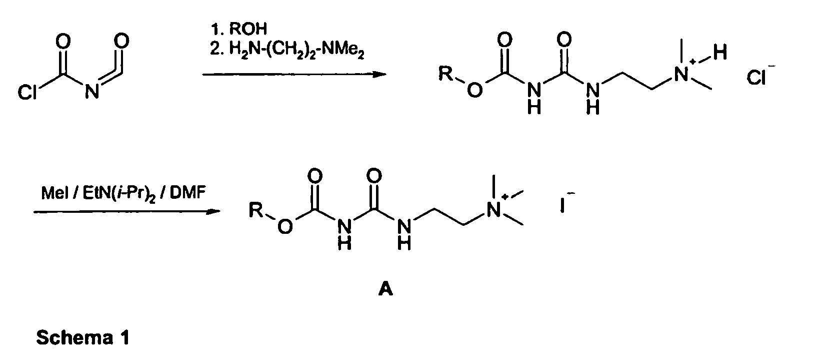

### Allgemeine Vorschrift (1) zur Synthese von Allophansäureester-Hydrochloriden

Zu einer Lösung von Chlorcarbonylisocyanat (0.76 mL, 9.5 mmol) in absolutem Diethylether (50 mL) wurde bei -50 °C unter Argon eine Lösung des entsprechenden Alkohols ROH (9.5 mmol) in absolutem Diethylether (50 mL) langsam zugetropft. Die Reaktionslösung wurde 1 Stunde (h) bei -50 °C gerührt und danach innerhalb einer Stunde auf 0 °C aufwärmt. Zum Reaktionsgemisch wurde anschließend bei 0 °C eine Lösung von *N,N*-Dimethylethylendiamin (9.5 mmol) und Triethylamin (1 mL) in absolutem Diethylether (50 mL) langsam zugetropft. Das Reaktionsgemisch wurde 16 h bei Raumtemperatur gerührt, mit Chlorwasserstoff in Diethylether angesäuert und 1 h bei Raumtemperatur gerührt. Der entstandene Feststoff wurde abfiltriert, mit Diethylether gewaschen und in Vakuo getrocknet. Der weiße Feststoff wurde in Wasser suspendiert. Die Suspension wurde auf zwei Zentrifugengläser verteilt, 2 × 20 min bei 5000 U/min zentrifugiert und der Überstand danach abdekantiert. Der isolierte amorphe Feststoff wurde i. Vak. über Phosphorpentoxid getrocknet.

### 4-(N,N-Dimethyl-3-aminoethyl)allophansäureoctylester-Hydrochlorid (OG-345.3)

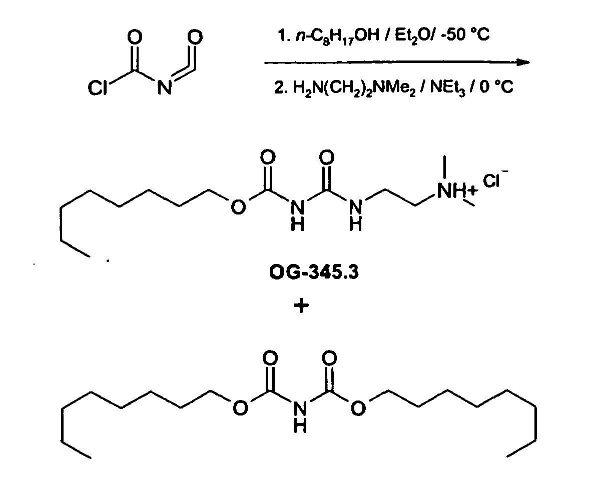

Die Synthese wurde nach der allgemeinen Vorschrift (1) durchgeführt.

Ansatzgröße: Chlorcarbonylisocyanat (0.76 mL, 9.5 mmol); 1-Octanol (1.53 mL, 9.5 mmol), *N,N*-Dimethylethylendiamin (1.05 mL, 840 mg, 9.5 mmol), Triethylamin (1 mL) in Diethylether (120 mL)
Ausbeute: 940 mg (31 %), weißer Feststoff
Schmelzpunkt: 86-92 °C
¹H-NMR (DMSO-d₆): 0.86 (3 H, t, *J* = 6.8 Hz); 1.20-1.35 (10 H, m); 1.57 (2 H, ddd, *J* = 6.8 + 6.8 + 13.7 Hz); 2.58 (6 H, s); 2.92 (2 H, t, *J* = 5.9 Hz); 3,43 (2 H, dd, *J* = 5.9 + 11.7 Hz); 4.05 (2 H, t, *J* = 6.8 Hz); 7.96 (1 H, t, *J* = 5.9 Hz); 10.00 (1 H, s).
¹³C-NMR (DMSO-d₆): 13.94; 22.04; 25.15; 28.18; 28.53 (2 C); 31.13; 35.12; 43.04 (2 C); 56.35; 64.95; 152.79; 153.98.

### 4-(N,N-Dimethyl-3-aminoethyl)allophansäuredodecylester-Hydrochlorid (OG-350)

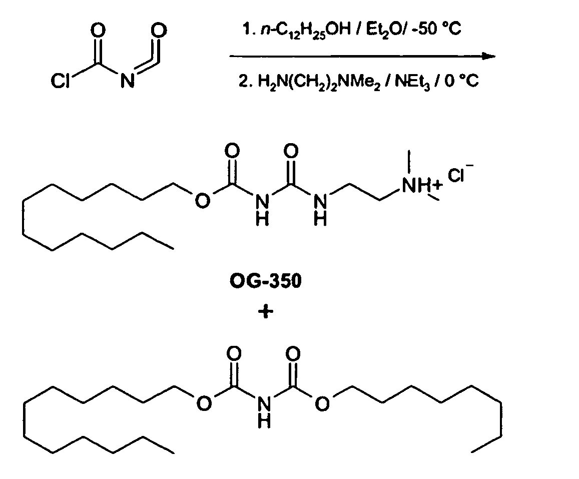

Die Synthese erfolgte nach der allgemeinen Vorschrift (1).

Ansatzgröße: Chlorcarbonylisocyanat (0.76 mL, 9.5 mmol), 1-Dodecanol (2.2 mL, 9.5 mmol); *N,N*-Dimethylethylendiamin (1.05 mL, 840 mg, 9.5 mmol), Triethylamin (1 mL) in Diethylether (120 mL).
Ausbeute: 410 mg (13 %), weißer Feststoff
Schmelzpunkt : 115-117 °C
¹H-NMR (DMSO-d₆): 0.80-0.90 (3 H, m); 1.16-1.36 (18 H, m); 1.50-1.64 (2 H, m); 2.77 (6 H, s); 3.17 (2 H, t, *J* = 5.5 Hz); 3.51 (2 H, dd, *J* = 6.3 + 11.7 Hz); 4.00-4.10 (2 H, m); 8.01 (1 H, t, *J* = 6.6 Hz); 10.07 (1 H, s).
¹³C-NMR (DMSO-d₆): 13.94; 22.06; 25.15; 28.18; 28.58; 28.66; 28.91 (2 C); 28.97 (2 C); 31.25; 34.47; 42.36 (2 C); 55.94; 64.99; 153.02; 153.95.

### Allgemeine Synthesevorschrift für die Quarternisierung der Allophansäureester-Hydrochloride (V2):

Ein Gemisch von Allophansäureester-Hydrochlorid (1 mmol) in absolutem N,N-Dimethylformamid (20 mL) und Ethyldiisopropylamin (340µL, 260 mg, 2 mmol) wurde mit Methyliodid (310 µL, 710 mg, 5 mmol) versetzt und anschließend 2.5 h bei 50 °C gerührt. Das Reaktionsgemisch wurde i. Vak. zur Trockne eingeengt, der Rückstand in Dichlormethan aufgenommen, erneut eingeengt und dann i. Vak. getrocknet. Der Rückstand wurde mit Wasser (80 mL) versetzt und gerührt. Die resultierende Suspension wurde zentrifugiert (20 min bei 5000 U/min) und danach wurde der Überstand abdekantiert. Der isolierte amorphe Feststoff wurde i. Vak. über Phosphorpentoxid getrocknet.

### Beispiel 1:

### 4-(N,N,N-Trimethyl-3-ammonio-ethyl)allophansäuredodecylester-Iodid (OG-351)

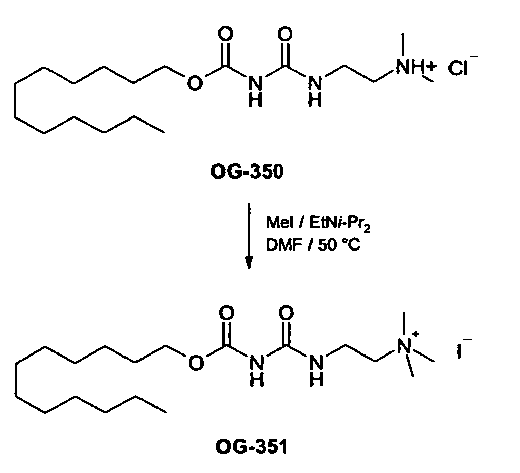

Die Synthese erfolgte nach der allgemeinen Vorschrift (V2).
Ansatzgröße: **OG-350** (330 mg, 0.87 mmol), Ethyldiisopropylamin (306 µL, 233 mg, 1.8 mmol) und Methyliodid (270 µL, 620 mg, 4.4 mmol) in *N,N*-Dimethylformamid (20 mL).
Ausbeute: 410 mg (96 %), weißer Feststoff
Schmelzpunkt: 138-146 °C
¹H-NMR (DMSO-d₆): 0.86 (3 H, t, *J* = 6.3 Hz); 1.10-1.36 (18 H, m); 1.50-1.64 (2 H, m); 3.10 (9 H, s); 3.42 (2 H, t, *J*= 6.3 Hz); 3.58-3.64 (2 H, m); 4.06 (2 H, t, *J* = 6.3 Hz); 8.09 (1 H, t , *J* = 6.3 Hz); 10.13 (1 H, s).
¹³C-NMR (DMSO-d₆): 13.93; 22.05; 25.12; 28.14; 28.55; 28.64; 28.89 (2 C); 28.95 (2 C); 31.22; 33.72; 52.48; 64.02; 65.06; 152.79; 154.05.

### Beispiel 2:

### Stufe 1:

### 4-(Pyrrolidyl-1-ethyl)allophansäurehexadecylester-Hydrochlorid (OG-358)

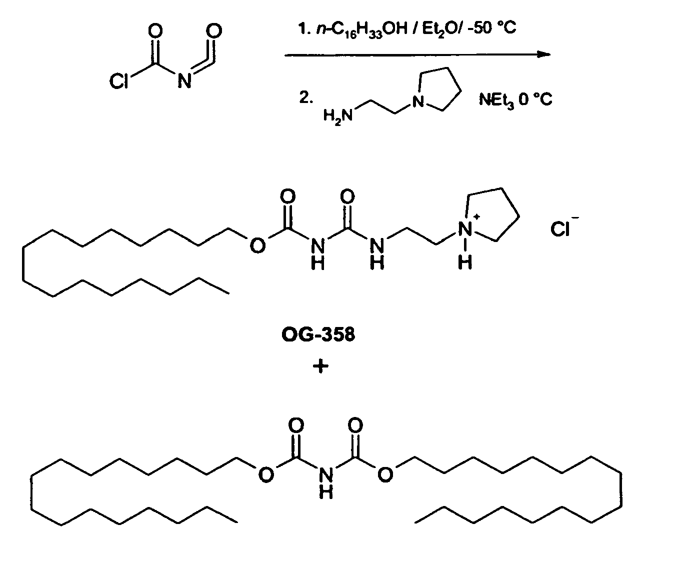

Die Synthese erfolgte nach der allgemeinen Vorschrift (V1):
Ansatzgröße: Chlorcarbonylisocyanat (0.76 mL, 9.5 mmol), 1-Hexadecanol (2.30 g, 9.5 mmol), 2-Pyrrolidin-1-yl-ethylamin (1.10 g, 9.5 mmol) und Triethylamin (1 mL) in Diethylether (150 mL).
Ausbeute: 580 mg (14 %), weißer Feststoff
Schmelzpunkt: 105-108 °C
¹H-NMR (DMSO-d₆ + CDCl₃): 0.84 (3 H, t, *J* = 7.0 Hz); 1.13-1.35 (26 H, m); 1.58 (2 H, ddd, *J* = 7.0 + 14.1 + 14.1 Hz); 1.80-2.10 (4 H, m); 3.0 (1 H, br. s); 3.08 (2 H, dd, *J* = 7.0 + 14.1 Hz); 3.20-3.40 (2 H, m); 3.52 (2 H, dd, *J* = 6.3 + 11.7 Hz); 3.59 (1 H, br. s); 4.0-4.12 (2 H, m); 8.06 (1 H, t, *J* = 5.8 Hz); 9.99 (1 H, s).

### Stufe 2:

### 4-(N-Methylpyrrolidinium-1-ethyl)allophansäurehexadecylester-Iodid (OG-359)

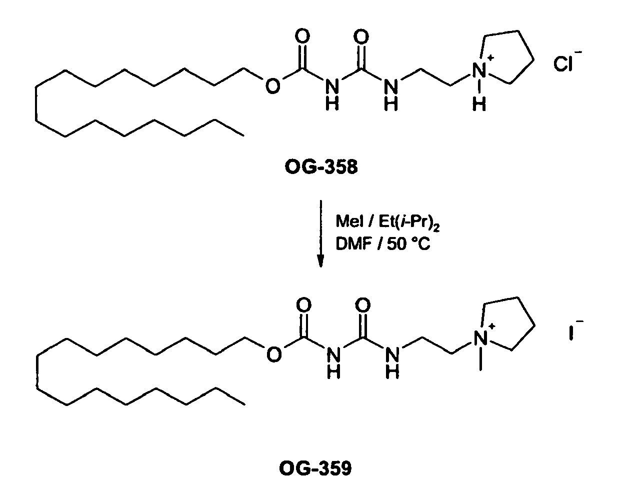

Die Synthese wurde nach der allgemeinen Vorschrift (V2):

Ansatzgröße: **OG-358** (480 mg, 1 mmol), Ethyldiisopropylamin (340 µL, 260 mg, 2 mmol) und Methyliodid (310 µL, 710 mg, 5 mmol) in *N,N*-Dimethylformamid (20 mL).
Ausbeute: 530 mg (93 %), weißer Feststoff
Schmelzpunkt : 107-110 °C
¹H-NMR (DMSO-d₆ + CDCl₃): 0.85 (3 H, t, *J* = 7.2 Hz); 1.14-1.40 (26 H, m); 1.50-1.63 (2 H, m); 2.92 (4 H, br. s); 3.05 (3 H, s); 3.42-3.67 (8 H, m); 4.06 (2 H, t, *J* = 5.5 Hz); 8.10 (1 H, t, *J* = 5.8 Hz); 10.09 (1 H, s).
¹³C-NMR (DMSO-d₆ + CDCl₃): 13.85; 20.91 (2 C); 22.02; 25.10; 28.13; 28.55; 28.61; 28.88; 28.95; 31.20; 34.20; 47.44; 61.69; 63.70 (2 C); 64.98; 152.78; 153.95.

### Beispiel 3:

### Stufe 1:

### 4-(N,N-Dimethyl-3-aminopropyl)allophansäurehexadecylester-Hydrochlorid (OG-361)

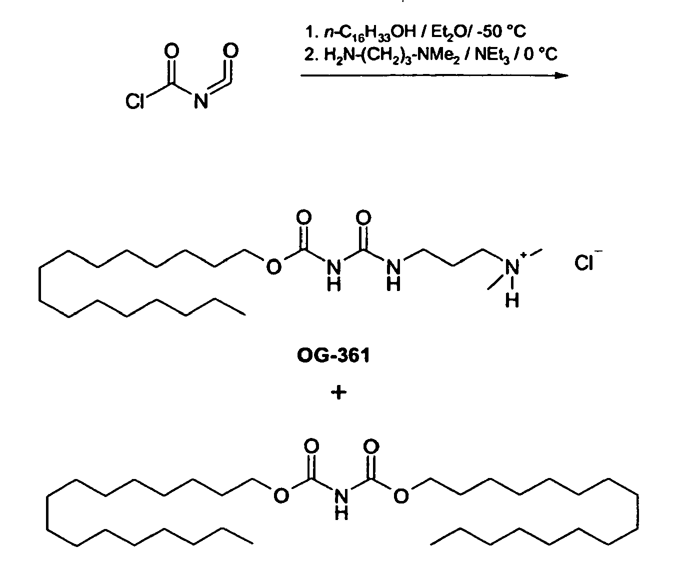

Die Synthese wurde nach der allgemeinen Vorschrift (V1) durchgeführt.
Ansatzgröße: Chlorcarbonylisocyanat (0.76 mL, 9.5 mmol), 1-Hexadecanol (2.30 g, 9.5 mmol), *N¹,N¹*-Dimethylpropan-1,3-diamin (1.2 mL, 970 mg, 9.5 mmol) und Triethylamin (1 mL) in Diethylether(150 mL).
Ausbeute: 1.70 g (40 %), weißer Feststoff
Schmelzpunkt: 120-123 °C
¹H-NMR (CDCl₃): 0.85 (3 H, t, *J* = 7.04 Hz); 1.20-1.30 (26 H, m); 1.62 (2 H, ddd, *J* = 7.0 + 7.0 + 14.1 Hz); 2.02-2.12 (2 H, m); 2.85 (6 H, s); 3.10-3.16 (2 H, m); 3.38 (2 H, dd, *J* = 6.3 + 12.5 Hz); 4.09 (2 H, t, *J* = 7.0 Hz); 7.93 (1 H, s); 7.98 (1 H, t, *J* = 6.3 Hz).

### Stufe 2:

### 4-(N,N,N-Trimethyl-3-ammonio-propyl)allophansäurehexadecylester-Iodid (OG-362)

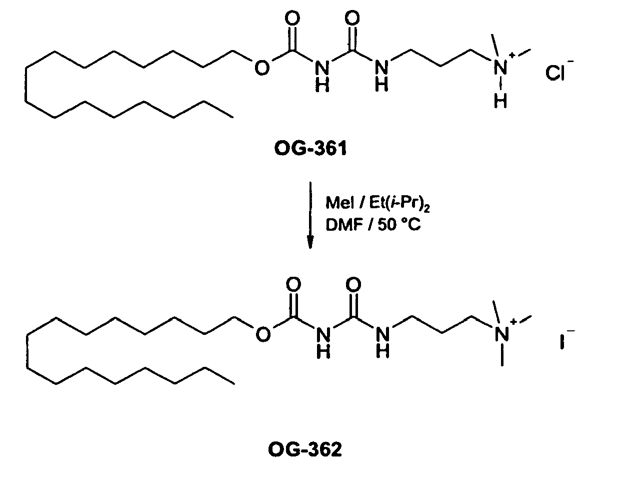

Die Synthese wurde nach der allgemeinen Vorschrift V2 durchgeführt.
Ansatzgröße: **OG-361** (450 mg, 1 mmol), Ethyldiisopropylamin (340 µL, 260 mg, 2 mmol) und Methyliodid (310 µL, 710 mg, 5 mmol) in *N,N*-Dimethylformamid (20 mL).
Ausbeute: 530 mg (95 %), weißer Feststoff
Schmelzpunkt: 153-154 °C (Umwandlung der Kristallform bei 115-116 °C).
¹H-NMR (DMSO-d₆): 0.80-0.90 (3 H, m); 1.16-1.40 (26 H, m); 1.57 (2 H, ddd, *J* = 6.8 + 6.8 + 13.7 Hz); 1.85-1.94 (2 H, m); 3.05 (9 H, s); 3.21 (2 H, dd, *J* = 6.8 + 12.7 Hz); 3.26-3.34 (2 H, m); 4.05 (2 H, t, *J* = 6.8 Hz); 7.90 (1 H, t, *J* = 5.9 Hz); 10.00 (1 H, s). ¹³C-NMR (DMSO-d₆): 13.91; 22.03; 23.16; 25.13; 28.16; 28.56-28.97 (10 C); 31.22; 36.30; 52.14; 63.24; 64.94; 152.69; 154.11.
Im aliphatischen Bereich sind Signale überlagert.

### Beispiel 4:

### Stufe 1:

### 4-(Dimethylamino-1-ethyl)allophansäureethylester (US-646)

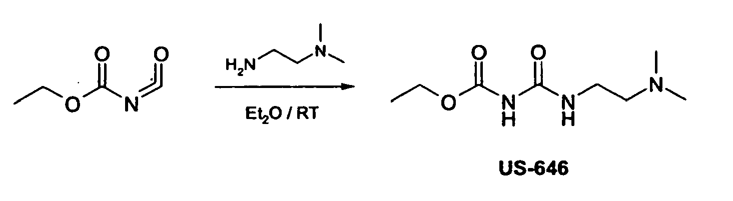

Eine Lösung von Isocyanatameisensäureethylester (500 mg, 4.2 mmol) in absolutem Diethylether (15 mL) wurde zu einer Lösung von *N¹,N¹*-Dimethylethan-1,2-diamin (440 mg, 550 µL, 5 mmol) in Diethylether (25 mL) getropft und 2 h bei Raumtemperatur gerührt. Der ausgefallene Niederschlag wurde abfiltriert und das Filtrat i.
Vak. eingeengt.
Ausbeute: 772 mg (90 %), weißer Feststoff
Schmelzpunkt: 40-67 °C
¹H-NMR (DMSO-d₆): 1.19 (3 H, t, *J* = 6.8 Hz); 2.13 (6 H, s); 2.31 (2 H, t, *J* = 6.3 Hz); 3.21 (2 H, q, *J* = 5.8 Hz); 4.09 (2 H, q, *J* = 6.8 Hz); 7.91 (1 H, t, *J* = 4.8 Hz); 9.70-10.10 (1 H, br s).

### Stufe 2:

### 4-Trimethylammonio-1-ethyl)allophansäureethylester-Iodid (US-647)

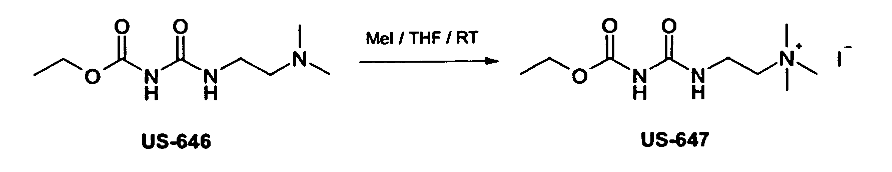

Eine Lösung von **US-646** (340 mg, 1.67 mmol) in absolutem Tetrahydrofuran (20 mL) wurde mit Methyliodid (1.18 g, 500 µL, 8.4 mmol) versetzt und 4 h bei Raumtemperatur gerührt. Der ausgefallene Niederschlag wurde abfiltriert und mit Tetrahydrofuran gewaschen.
Ausbeute: 530 mg (92 %), weißer Feststoff
Schmelzpunkt: 172-175 °C
¹H-NMR (DMSO-d₆): 1.20 (3 H, t, *J* = 7.2 Hz); 3.10 (9 H, s); 3.42 (2 H, t, *J* = 5.8 Hz); 3.61 (2 H, m); 4.11 (2 H, q, *J* = 6.8 Hz); 8.12 (1 H, t, *J* = 5.8 Hz); 10.19 (1 H, s). ¹³C-NMR (DMSO-d₆): 14.2; 33.7; 52.5 (3 C); 61.9; 64.1; 153.1; 154.3.

### Beispiel 5:

### Synthese von 4-(N,N,N-Trimethylammonioethyl)allophansäure-(3-hexadecyloxy-2-methoxypropan-1-yl-ester-Iodid

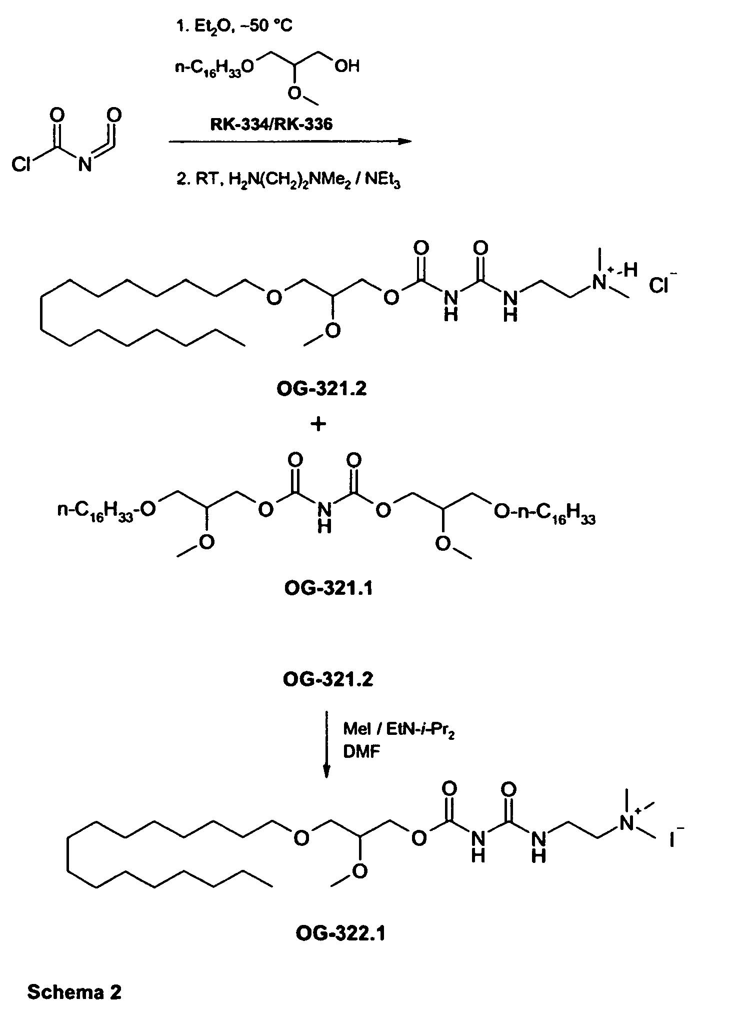

### Stufe 1:

### cis-5-Methoxy-2-phenyl-[1,3]dioxan (RK-330)

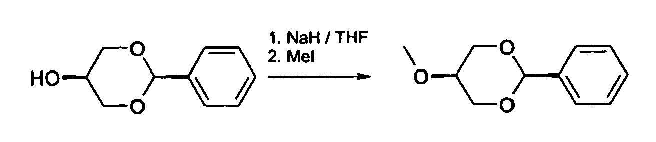

Zu einem Gemisch von 55 % öliger Natriumhydrid-Suspension (1.96 g, 45 mmol) in wasserfreiem Tetrahydrofuran wurde eine Lösung von cis-1,3-Benzylidenglycerol (5.47 g, 30 mmol) in wasserfreiem Tetrahydrofuran (50 mL) getropft. Nach Beendigung der Wasserstoffentwicklung wurde unter Eiskühlung eine Lösung von Methyliodid (8.52 g, 3.74 mL, 60 mmol) in wasserfreiem Tetrahydrofuran (20 mL) zugefügt und danach bei Raumtemperatur über Nacht gerührt. Das Reaktionsgemisch wurde filtriert, das Filtrat i. Vak. zur Trockne eingeengt und der Rückstand in Toluol aufgenommen. Die Toluol-Lösung wurde filtriert, der Filterrückstand mit Toluol (25 mL) gewaschen und das Filtrat i. Vak. eingeengt. Nach Kristallisation des Rückstandes (gelber Feststoff) wurde das Weißöl abdekantiert.
Ausbeute: 5.57 g (95 %)
¹H-NMR (CDCl₃): 3.18 (1 H, q, *J* = 2 Hz); 3.48 (3 H, s); 4.05 (2 H, dd, *J* = 2 und 12.5 Hz); 4.37 (2 H, d, *J* = 12.5 Hz); 5.56 (1 H, s); 7.31-7.37 (4 H, m); 7.50-7.53 (1 H, m).

### Stufe 2:

### 2-Methoxypropan-1,3-diol (RK-331)

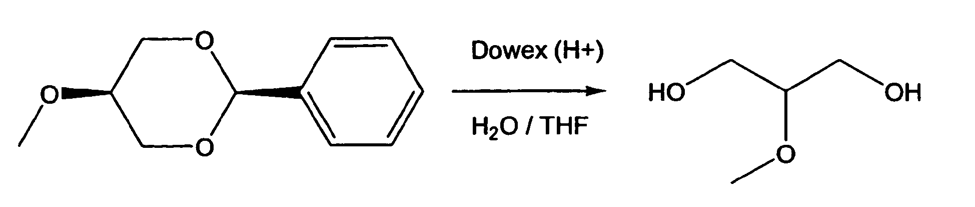

Eine Lösung von **RK-330** (5.55 g, 28 mmol) in einem Gemisch aus Wasser (10 mL) /Tetrahydrofuran (20 mL) wurde mit stark saurem Dowex-Ionenaustauscher (2 g) versetzt und 5 h unter Rückfluss erhitzt. Das Reaktionsgemisch wurde anschließend, filtriert und der Filterrückstand mit destilliertem Wasser (25 mL) gewaschen. Das Filtrat wurde i. Vak. eingeengt und der Rückstand in Wasser (100 mL) aufgenommen. Die wässrige Phase wurde mit tert-Butyl-methylether (3 × 25 mL) extrahiert. Die wässrige Phase wurde i. Vak. eingeengt und der Rückstand zur azeotropen Trocknung in Pyridin (3×10 mL) gelöst und jeweils eingeengt.
Ausbeute: 2.60 g (88 %), farblose Flüssigkeit
¹H-NMR (DMSO-d6): 3.12 (1 H, dt, *J* = 4.2, 10.4 Hz); 3.32 (3 H, s); 3.34-3.47 (4 H, m); 4.45 (2 H, t, *J* = 6 Hz).

### Stufe 3:

### 3-Hexadecyloxy-2-methoxy-propan-1-ol (RK-334)

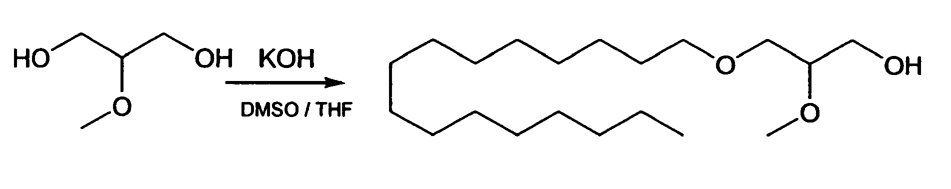

Eine Lösung von **RK-331** (2.60 g, 24.5 mmol) in wasserfreiem Dimethylsulfoxid (20 mL) und wasserfreiem Tetrahydrofuran (20 mL) wurde mit gepulvertem Kaliumhydroxid (1.4 g, 25 mmol) versetzt Anschließend wurde eine Lösung von lodhexadecan (4.58 g, 13 mmol) in wasserfreiem Tetrahydrofuran (20 mL) zugetropft und die Mischung 16 h bei Raumtemperatur gerührt. Die Suspension wurde i. Vak. eingeengt, der Rückstand mit Wasser (40 mL) versetzt und mit tert-Butyl-methylether (3 × 50 mL) extrahiert. Die vereinigten organischen Phasen wurden mit Natriumsulfat getrocknet, filtriert und zur Trockne eingeengt. Der Rückstand wurde durch Flashchromatographie (100 g, 20 × 4 cm) mit tert-Butyl-methylether / Cyclohexan (2:1) gereinigt.
Ausbeute: 2.00 g (46 % bezogen auf Iodhexadecan), farbloser Feststoff Schmelzpunkt: 31-33 °C
¹H-NMR (CDCl₃): 0.88 (3 H, t, *J* = 7.2 Hz); 1.22-1.35 (26 H, m); 1.57 (2 H, 7.1 Hz); 2.14 (1 H, dd., *J* = 5.4 und 7.0 Hz); 3.40-3.48 (1H, m); 3.47 (3 H, s); 3.48-3.58 (2 H, m); 3.61-3.68 (1 H, m); 3.73-3.79 (1 H, m).

### Stufe 4:

### 4-(N,N-Dimethylaminoethyl)allophansäure-3-hexadecyloxy-2-methoxypropan-1-yl-ester-Hydrochlorid (OG-321.2) und Imidodicarbonsäuredi-3-hexadecyloxy-2-methoxypropan-1-yl-ester (OG-321.1)

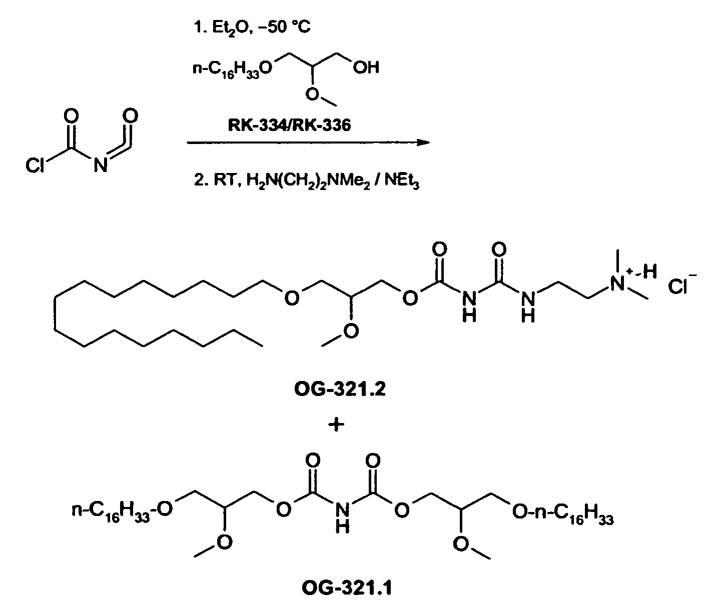

Zu einer Lösung von Chlorcarbonylisocyanat (0.76 mL, 9.5 mmol) in absolutem Diethylether (50 mL) wurde bei -50 °C unter Argon eine Lösung von **RK-334** (3.14 g, 9.5 mmol) in absolutem Diethylether (40 mL) langsam zugetropft. Die Reaktionslösung wurde 1 h unter Argon bei -60 °C gerührt und danach innerhalb einer Stunde auf 0 °C aufwärmt. Zum Reaktionsgemisch wurde anschließend bei 0 °C eine Lösung von *N,N*-Dimethylethylendiamin (1.05 mL, 840 mg, 9.5 mmol) und Triethylamin (1.0 mL) in absolutem Diethylether (50 mL) langsam zugetropft. Das Reaktionsgemisch wurde bei Raumtemperatur 16 h gerührt, mit Chlorwasserstoff in Diethylether angesäuert und 1 h bei Raumtemperatur gerührt. Der entstandene Feststoff wurde abfiltriert, mit Diethylether gewaschen und i. Vak. getrocknet. Der weiße Feststoff wurde in Wasser (100 mL) suspendiert. Die Suspension auf zwei Zentrifugengläser verteilt, danach 2x20 min bei 5000 U/min zentrifugiert und anschließend dekantiert. Der isolierte amorphe Feststoff (**OG-321.2**) wurde i. Vak. über Phosphorpentoxid getrocknet.

### OG-321.2

Ausbeute: 310 mg (7 %), weißer Feststoff
Schmelzpunkt: 59-63 °C
¹H-NMR (DMSO-d₆): 0.85 (3 H, t, *J* = 6.8 Hz); 1.18 -1.24 (26 H, m); 1.45-1.50 (2 H, m); 2.79 (6 H, s); 3.00-3.56 (9 H, m); 3.32 (3 H, s); 4.04 (1 H, dd, *J* = 5.6 und 11.6 Hz); 4.21 (1 H, dd, *J* = 3.6 und 11.2 Hz); 7.98 (1 H, br.t, *J* = 5.9 Hz); 9.70 (1 H, br s); 10.17 (1 H, s).
¹³C-NMR (DMSO-d₆): 13.94; 22.05; 25.55; 28.64; 28.77; 28.96; 29.06; 31.23; 34.53; 38.88; 42.45 (2 C); 56.08; 57.04; 64.15; 68.74; 70.53; 77.21; 152.93; 153.75.
Im aliphatischen Bereich sind Signale überlagert.

Die vereinigten Diethylether-Filtrate wurden i. Vak. eingeengt und getrocknet, wodurch **OG-321.1** isoliert wurde.

### OG-321.1

Ausbeute: 920 mg (52 %), weißer Feststoff
Schmelzpunkt: 40-42 °C
¹H-NMR (DMSO-d₆ + CDCl₃): 0.81 (6 H, t, *J* = 6.8 Hz); 1.10-1.30 (55 H, m); 1.40-1.54 (4 H, m); 2.80-3.55 (6 H, m); 3.34 (6 H, s); 4.01 (1 H, d, *J* = 6.8 Hz); 4.04 (1 H, d, *J* = 5.8 Hz); 4.18 (1 H, dd, *J* = 2.0 und 3.9 Hz); 4,21 (1 H, dd, *J* = 2.0 und 3.9 Hz); 10.20 (1 H, s).

### Stufe 5:

### 4-(N,N,N-Trimethylammonioethyl)allophansäure-3-hexadecyloxy-2-hexadecyloxy-1-yl-ester-Iodid (OG-322.1)

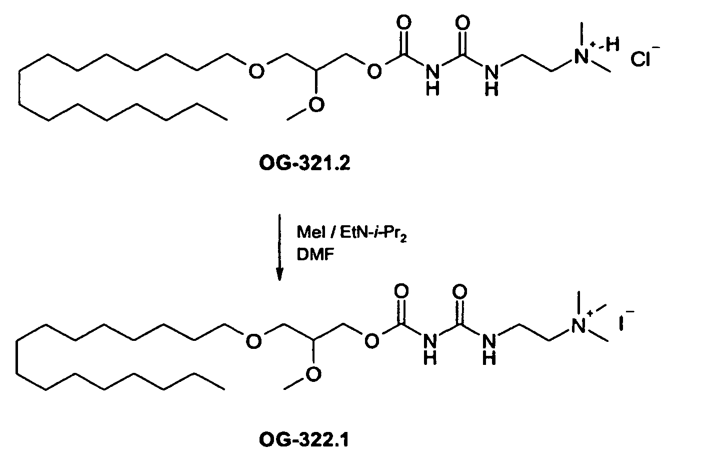

Eine Mischung von **OG-321.1** (210 mg, 0.40 mmol) in absolutem *N,N*-Dimethylformamid (10 mL) und Ethyldiisopropylamin (136 µL, 103 mg, 0.80 mmol) wurde mit Methyliodid (125 µL, 280 mg, 2 mmol) versetzt und anschließend 2.5 h bei 50°C gerührt. Das Reaktionsgemisch wurde i. Vak. zur Trockne eingeengt, der Rückstand in Dichlormethan aufgenommen, erneut eingeengt und dann i. Vak. getrocknet. Der Rückstand (400 mg) wurde mit Wasser (25 mL) versetzt und 30 min bei Raumtemperatur gerührt. Die Suspension wurde zentrifugiert (20 min bei 5000 U/min) und danach dekantiert. Der isolierte amorphe Feststoff wurde in Methanol aufgenommen, die resultierende Mischung eingeengt und der Rückstand i. Vak. über Phosphorpentoxid getrocknet.
Ausbeute:120 mg (48 %), weißer Feststoff
Schmelzpunkt: 136-141 °C
¹H-NMR (DMSO-d₆): 0.80-0.90 (3 H, m); 1.20-1.35 (26 H, m); 1.40-1.50 (2 H, m); 3.10 (9 H, s); 3.32 (3 H, s); 3.20-3.64 (9 H, m); 4.05 (1 H, dd, *J* = 5.2 und 11.2 Hz); 4.22 (1 H, dd, *J* = 5.2 und 11.2 Hz); 8.06 (1 H, br t, *J* = 5.9 Hz); 10.22 (1 H, s).
¹³C-NMR (DMSO-d₆): 13.93; 22.04; 25.54; 28.64; 28.77; 28.96; 29.05; 31.22; 33.76; 52.47 (3 C); 57.04; 63.99; 64.19; 68.73; 70.52; 77.19; 152.67; 153.84.
Im aliphatischen Bereich sind Signale überlagert.

### Beispiel 6:

### Synthese von 4-(N,N,N-Trimethylammoniethyl)allophansäure-(cis-5-hexa-decyloxymethyltetrahydrofuran-2-yl)methylester-Iodid

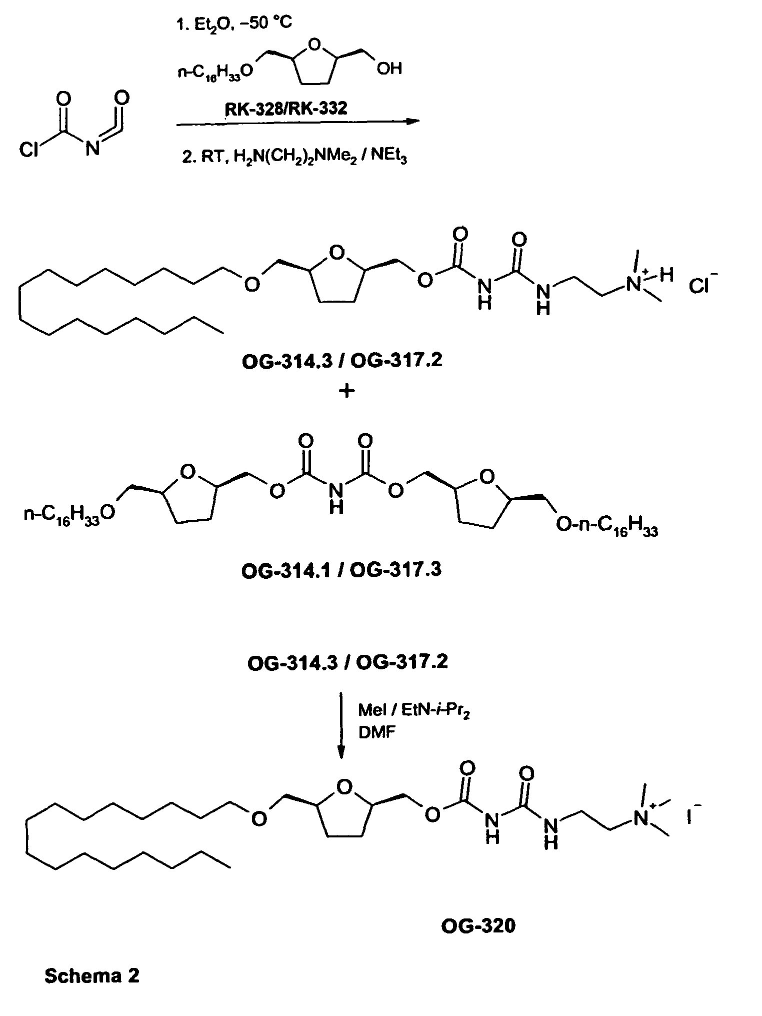

### Stufe 1:

### Synthese von (5-Hydroxymethylfuran-2-yl)methanol (RK-325)

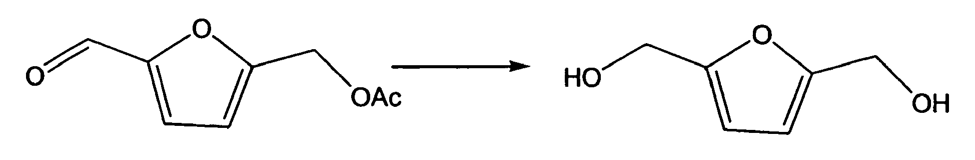

Eine Lösung von Essigsäure5-formylfuran-2-yl-methylester (5.05 g, 30 mmol) in absolutem Ethanol (150 mL) wurde bei 0°C mit Natriumborhydrid (1.2 g, 32 mmol) versetzt. Anschließend wurde auf Raumtemperatur erwärmt und über 48 Stunden gerührt. Dann wurde auf 0 °C abgekühlt und mit konzentrierter Salzsäure auf pH 4 angesäuert und danach sofort mit 5 % Natriumhydrogencarbonat-Lösung neutralisiert. Das Gemisch wurde filtriert und das Filtrat i. Vak. zur Trockne eingeengt. Der Rückstand wurde in Ethanol (30 mL) aufgenommen, filtriert und das Filtrat zur Trockne eingeengt.

Ausbeute 3.48 g (91 %); schwach braunes Öl
¹H-NMR (CDCl₃): 4.58 (4 H, s); 6,23 (2 H, s);

### Stufe 2:

### Synthese von (5-Hydroxymethyltetrahydrofuran-2-yl)methanol (RK-325)

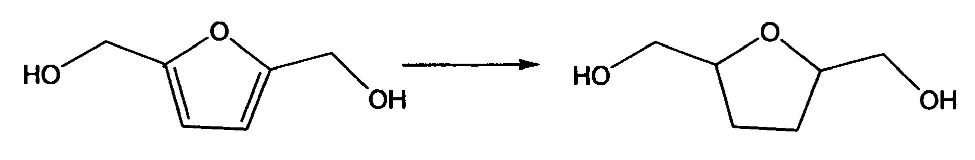

Eine Lösung von (5-Hydroxymethylfuran-2-yl)methanol (RK-325) (3.46 g, 27 mmol) in absolutem Ethanol (100 mL) wurde mit Raney-Nickel [50 % käufliche wässrige Raney-Nickel-Suspension (5mL) von Acros, wurde dreimal mit Ethanol versetzt und dekantiert] versetzt und über Nacht bei Raumtemperatur und einem Druck von 2 bar hydriert (Wasserstoffverbrauch 61 mmol). Das Reaktionsgemisch wurde filtriert und das Filtrat i. Vak. eingeengt.

Ausbeute 3.17 g (89%); farblose Flüssigkeit
¹H-NMR (CDCl₃): 1.78-2.00 (4H, m); 3.54 (2H, dd, J = 5.2 und 11.2 Hz); 3.79 (2H, dd, J = 1.6 und 11.2 Hz), 4.07-4.14 (2H, m).

### Stufe 3:

### Synthese von (5-Hexadecyloxymethyltetrahydrofuran-2-yl)methanol (RK-328)

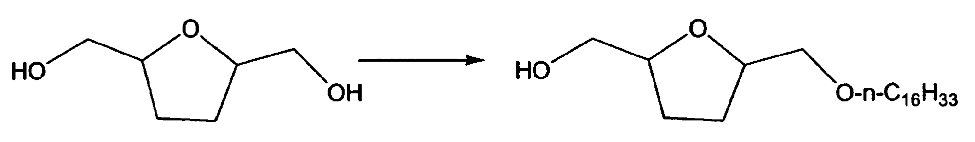

Eine Lösung von RK-325 (3.16 g, 23.9 mmol) in wasserfreim Dimethylsulfoxid (20 mL) und wasserfreiem Tetrahydrofuran (20 mL) wurde mit pulverisiertem Kaliumhydroxid (1.345 g, 24 mmol) versetzt. Zu dieser Suspension wurde bei Raumtemperatur eine Lösung von 1-lodhexadecan (3.52 g, 10 mmol) in wasserfreiem Tetrahydrofuran (20 mL) getropft. Anschließend wurde 16 Stunden bei Raumtemperatur gerührt. Die Suspension wurde i. Vak. eingeengt und der Rückstand mit Wasser (40 mL) versetzt. Dananch wurde mit tert-Butylmethylether (3 x 50 mL) extrahiert. Die vereinigten organischen Extrakte wurden mit Natriumsulfat getrocknet, filtriert und i. Vak. zur Trockne eingeengt. Der Rückstand wurde durch Flashchromatographie (100g, 23 x 4 cm) mit tert-Butylmethylether / Cyclohexan (2:1) gereinigt.
Ausbeute 1.76 g (49 % bezogen auf n-C16H33I); weißer Feststoff
Schmelzpunkt 29-30 °C
¹H-NMR (CDCl₃): 0.88 (3H, t, J = 6.8Hz); 1.25-1.38 (24 H, m); 1.59 (2H, dt, J = 2.8 und 7.0 Hz); 1.89-1.96 (4H, m); 2.69 (1 H, dd, J = 4.3 und 8.4 Hz); 3.40-3.51 (6H, m); 3.60 (1 H, dd, J = 3.0 und 10.2 Hz); 3.80 (1 H, ddd, J = 3.9, 4.1 und 12.4 Hz); 4.10 (2 H, m).

### Stufe 4:

### 2-(N,N-Dimethylaminoethyl)allophansäure-(cis-5-hexadecyloxymethyltetrahydrofuran-2-yl)methylester-Hydrochlorid (OG-317.2) und Imidodicarbonsäure-di(cis-5-hexadecyloxymethyltetrahydrofuran-2-yl) methylester (OG-317.3)

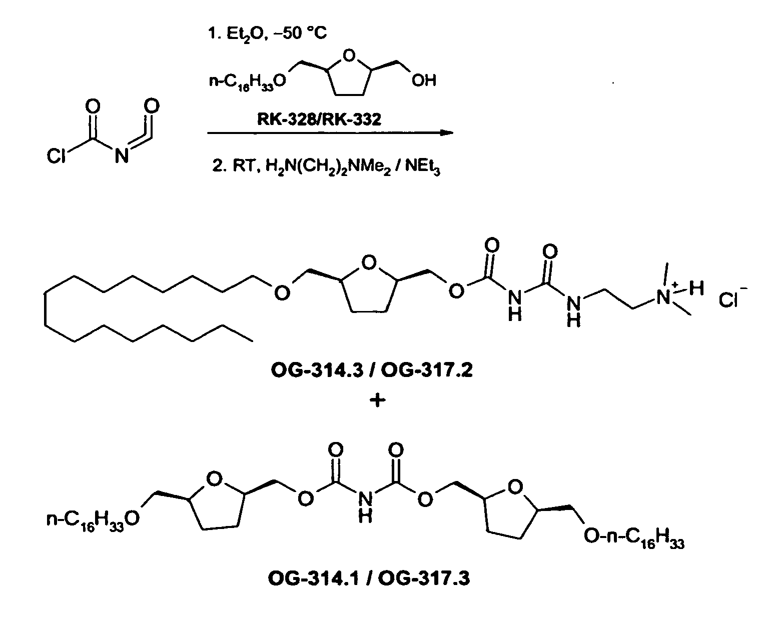

Zu einer Lösung von Chlorcarbonylisocyanat (0.40 mL, 5 mmol) in absolutem Diethylether (30 mL) wurde bei -50 °C unter Argon eine Lösung von **RK-332** (1.8 g, 5 mmol) in absolutem Diethylether (40 mL) langsam zugetropft. Die Reaktionslösung wurde 1 h bei -60 °C unter Argon gerührt und danach innerhalb einer Stunde auf 0 °C aufwärmt. Zum Reaktionsgemisch wurde anschließend bei 0 °C eine Lösung von *N,N*-Dimethylethylendiamin (0.55 mL, 440 mg, 5 mmol) und Triethylamin (0.5 mL) in absolutem Diethylether (30 mL) langsam zugetropft. Das Reaktionsgemisch wurde bei Raumtemperatur 16 h gerührt, danach mit Chlorwasserstoff in Diethylether angesäuert und 1 h bei Raumtemperatur gerührt. Der entstandene Feststoff wurde abgesaugt, mit Diethylether gewaschen und in Vak. getrocknet. Der weiße Feststoff (2.88 g) wurde in Wasser (100 mL) suspendiert. Die Suspension auf zwei Zentrifugengläser verteilt, 2 × 20 min bei 5000 U/min zentrifugiert und danach dekantiert. Der isolierte amorphe Feststoff (**OG-317.2**) wurde i. Vak. über Phosphorpentoxid getrocknet.

### OG-317.2

Ausbeute 500 mg (18 %), weißer Feststoff
Schmelzpunkt: 84-85 °C
¹H-NMR (DMSO-d₆): 0.86 (3 H, t, *J* = 6.6 Hz); 1.14-1.34 (26 H, m); 1.42-1.52 (2 H, m); 1.56-1.70 (2 H, m); 1.82-1.89 (2 H, m); 2.76 (6 H, s); 3.15 (2 H, dd, *J* = 5.5 und 5.5 Hz); 3.30-3.40 (4 H, m); 3.50 (2 H, dd, *J* = 6.2 und 11.7 Hz); 3.97 (2 H, dd, *J* = 6.4 und10.8 Hz); 4.00-4.08 (1 H, m); 4.11 (1 H, dd, *J* = 3.6 und 11.2 Hz); 7.99 (1 H, t, *J* = 5.5 Hz); 10.18 (1 H, s).
¹³C-NMR (DMSO-d₆): 13.94; 22.06; 25.61; 27.00; 27.50; 28.65-29.16 (11 C); 31.24; 34.60; 42.52; 56.12; 67.16; 70.42; 72.84; 76.21; 78.10; 152.97; 153.82.
Im aliphatischen Bereich sind Signale überlagert.

Die vereinigten Diethylether-Filtrate wurden eingeengt und i. Vak. getrocknet, wodurch **OG-317.3** isoliert wurde.

### OG-317.3

Ausbeute 610 mg (31 %), weißer Feststoff
Schmelzpunkt: 42-45 °C
¹H-NMR (DMSO-d₆ + CDCl₃): 0.80-0.88 (6 H, m); 1.14-1.34 (52 H, m); 1.42-1.52 (4 H, m); 1.58-1.72 (4 H, m); 1.80-2.00 (4 H, m); 3.26-3.40 (8 H, m); 3.90-4.16 (8 H, m); 10.48 (1 H, s).

### 4-(N,N,N-Trimethylammonioethyl)allophansäure-(cis-5-hexadecyloxymethyl tetrahydrofuran-2-yl)methylester-lodid (OG-320)

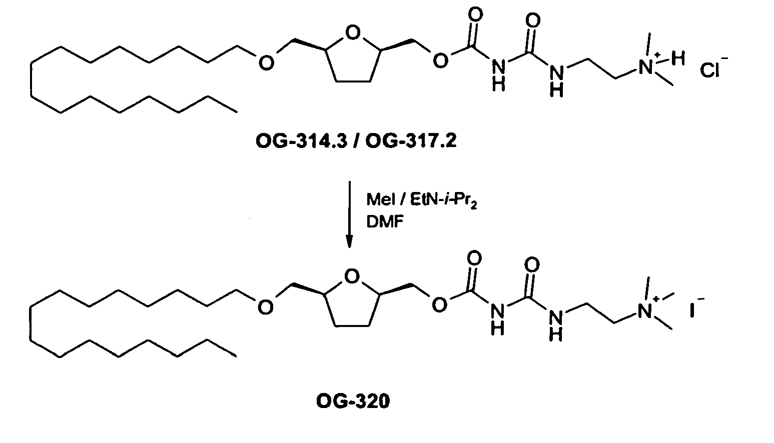

Eine Mischung von **OG-314.3/OG-317.2** (600 mg, 1.1 mmol) in absolutem *N,N*-Dimethylformamid (25 mL) und Ethyldiisopropylamin (370 µL, 2.2 mmol) wurde mit Methyliodid (340 µL, 775 mg, 5,5 mmol) versetzt und danach 2.5 h bei 50 °C gerührt. Das Reaktionsgemisch wurde i. Vak zur Trockne eingeengt, der Rückstand in Dichlormethan aufgenommen, die resultierende Suspension erneut eingeengt und der Feststoff i. Vak. getrocknet. Der Rückstand (1.33 g) wurde mit Wasser (80 mL) versetzt und bei Raumtemperatur 30 min gerührt. Die Suspension wurde zentrifugiert (20 min bei 5000 U/min) und danach dekantiert. Der isolierte amorphe Feststoff wurde mit Methanol versetzt, die Suspension eingeengt und der Rückstand anschließend i. Vak. über Phosphorpentoxid getrocknet.
Ausbeute: 620 mg (86 %), weißer Feststoff
Schmelzpunkt : 96-101 °C
¹H-NMR (DMSO-d₆): 0.85 (3 H, t, J = 6,4 Hz); 1.12 -1.36 (26 H, m); 1.40-1.50 (2 H, m); 1.50-1.68 (2 H, m); 1.80-2.00 (2 H, m); 3.10 (9 H, s); 3.15-3.50 (4 H, m); 3.54-3.64 (2 H, m); 3.97 (2 H, dd, J = 5.9 + 10.8 Hz); 4.00-4.08 (1 H, m); 4.12 (1 H, dd, J = 2.9 und 10.8 Hz); 8.07 (1 H, t, J = 5.8 Hz); 10.21 (1 H, s).
¹³C-NMR (DMSO-d₆):13.93; 22.06; 25.61; 27.00; 27.50; 28.65-29.16 (11 C); 31.24; 33.77; 52.46; 52.49; 52.53; 64.02; 67.20; 70.42; 72.84; 76.21; 78.09; 152.73; 153.89.
Im aliphatischen Bereich sind Signale überlagert.

### Beispiel 7:

### Synthese von 4-(N,N,N-Trimethylammonioethyl)allophansäure-(2-hexa-decyloxymethyltetrahydrofuran-2-yl)methylester-Iodid

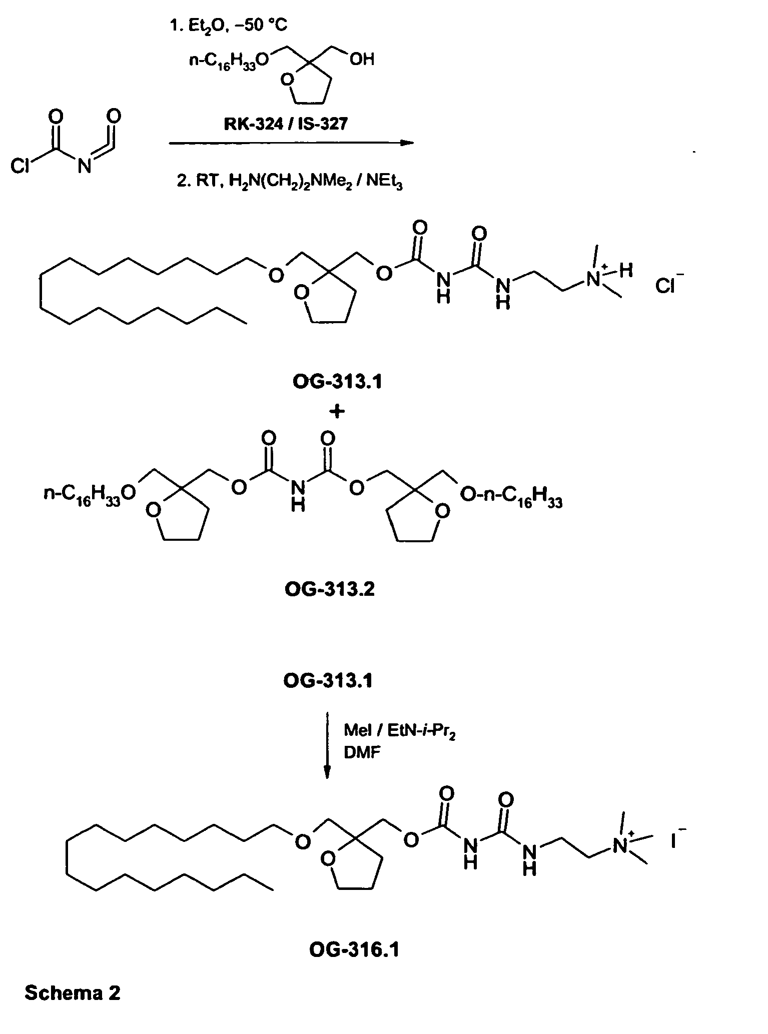

### Stufe 1:

### Tetrahydrofuran-2-carbonsäuremethylester (RK-321)

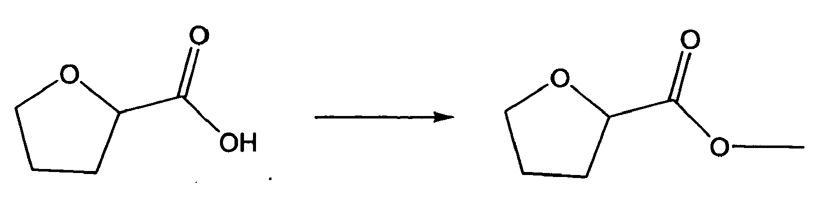

Eine Lösung von Tetrahydrofuran-2-carbonsäure (37.7 g, 325 mmol) in chlorwasserstoffhaltigem Methanol (200 mL9 wurde über Nacht bei Raumtemperatur gerührt. Das Lösungsmittel wurde i. Vak. entfernt und der Rückstand in Dichlormethan (150 mL) aufgenommen. Die Lösung wurde mit Natriumhydrogencarbonat-Lösung und Wasser (jeweils 2 x 100 mL) gewaschen, über Natriumsulfat getrocknet, filtriert und zur Trockne eingeengt.
Ausbeute: 32.7 g (78 %)
¹H-NMR (CDCl₃): 1.84-2.35 (4H, m); 3.74 (3H, s); 3.92 (1 H, dt, J = 5.6 und 7.2 Hz); 4.01 (1 H, dt, J = 6.0 und 8.4 Hz); 4.45 (1 H, dd, J = 5.6 und 8.4 Hz).

### Stufe 2:

### (2-Hydroxymethyltetrahydrofuran-2-yl)methanol (RK-322)

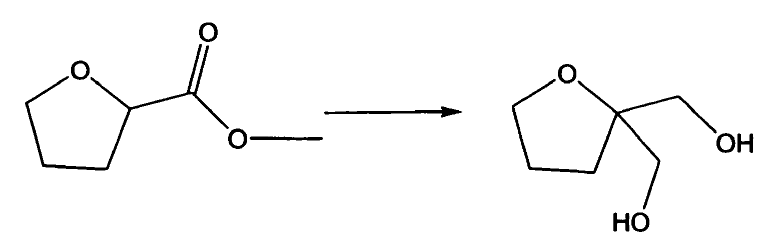

Eine Lösung von RK-321 (5.25 g, 40 mmol) in wasserfreiem Tetrahydrofuran (50 mL) wurde bei -78 °C mit einer 1.2 M Lösung von Diisobutylaluminiumhydrid in Toluol (45 mmol, 37.5 mL) versetzt, so dass die Innentemperatur nicht über -65 °C stieg. Anschließend wurde 90 min. bei -78 °C gerührt und dann mit wasserfreiem Methanol (6 mL) versetzt, so dass die Innentemperatur unter - 50 °C blieb. Danach wurde das Reaktionsgemisch langsam auf 5 °C erwärmt. Diese Lösung wurde zu einer gekühlten Lösung von Natriumhydroxid (13.0 g, 0.325 mmol) Wasser (45 mL) und 37 % Formalin (56.5 mL, 0.975 mmol) getropft, wobei die Innentemperatur unter 12 °C blieb. Nach 5 Min. wurde das Reaktionsgemisch mit Ameisensäure (1.7 mL) versetzt und auf 65 °c erwärmt. Danach wurden die Lösungsmittel i.Vak. abdestilliert. Der feste Rückstand wurde mit Dichlormethan (25 mL) versetzt und 15 Min. im Ultraschallbad behandelt. Die Suspension wurde filtriert und der Rückstand mit Dichlormethan (100 mL) gewaschen. Die vereinigten Filtrate wurden i.Vak. eingeengt und der Rückstand im Kugelrohr destiliert (Badtemperatur 140 °C, 0.14 mbar).
Ausbeute: 1.64 g (31 %); weißer Feststoff, der bei ca. 40 °C schmilzt.
¹H-NMR (CDCl₃): 1.78 (2H, dt, J = 0.8 und 7.6 Hz); 1.92-2.00 (5H, m); 3.55 (2H, br d, J = 10.8 Hz); 3.63 (2H, br d, J = 10.8 Hz); 3.89 (2H, t, J = 6.4 Hz).

### Stufe 3:

### (2-Hexadecyloxymethyl-tetrahydrofuran-2-yl)-methanol

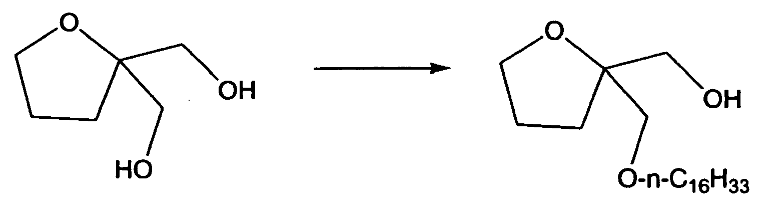

Eine Lösung von RK-322 (1.60 g, 12.2 mmol) in wasserfreim Dimethylsulfoxid (10 mL) und wasserfreiem Tetrahydrofuran (10 mL) wurde mit pulverisiertem Kaliumhydroxid (684 mg, 12.2 mmol) versetzt. Zu dieser Suspension wurde bei Raumtemperatur eine Lösung von 1-lodhexadecan (1.41 g, 4 mmol) in wasserfreiem Tetrahydrofuran (6 mL) getropft. Anschließend wurde 16 Stunden bei Raumtemperatur gerührt. Die Suspension wurde i. Vak. eingeengt und der Rückstand mit Wasser (20 mL) versetzt. Dananch wurde mit tert-Butylmethylether (3 x 25 mL) extrahiert. Die vereinigten organischen Extrakte wurden mit Natriumsulfat getrocknet, filtriert und i. Vak. zur Trockne eingeengt. Der Rückstand wurde durch Flashchromatographie (90g, 20 x 4 cm) mit tert-Butylmethylether / Cyclohexan (3:1) gereinigt.
Ausbeute 744 mg (51 % bezogen auf n-C16H33I); farbloses Öl
¹H-NMR (CDCl₃): 0.89 (3H, t, J = 7.2 Hz); 1.26 (26 H, br s); 1.52-1.59 (2H, m); 1.76-1.96 (4H, m); 2.21 (2H, t, J = 7.2); 3.34-3.62 (6H, m); 3.86 (2H, dt, J = 1.2 und 6.6 Hz).
¹³C-NMR (CDCl₃): 14.25; 22.82; 26.23; 29.48; 29.58; 29.71; 29.80; 30.65; 32.03; 66.60; 68.61; 72.07; 74.41; 83.96;

### Stufe 4:

### 4-(N,N-Dimethylaminoethyl)allophansäure-(2-hexadecyloxymethyl tetrahydrofuran-2-yl)methylester(OG-313.1) und Imidodicarbonsäure-di-(2-hexadecyloxymethyltetrahydrofuran-2-yl)methylester (OG-313.2)

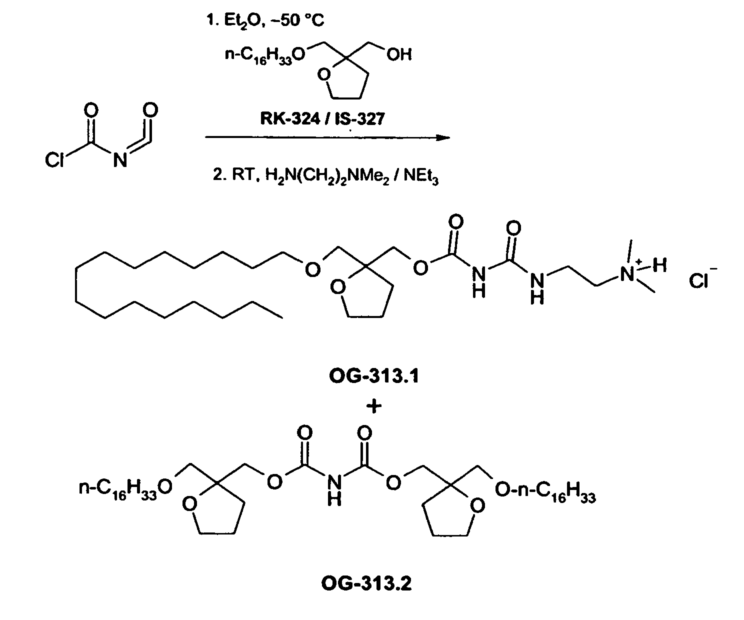

Zu einer Lösung von Chlorcarbonylisocyanat (0.40 mL, 5 mmol) in absolutem Diethylether (30 mL) wurde bei -50 °C unter Argon eine Lösung von **IS-320** (1.8 g, 5 mmol) in absolutem Diethylether (26 mL) langsam zugetropft. Die Reaktionslösung wurde 1 h bei -60 °C unter Argon gerührt und danach innerhalb einer Stunde auf 0°C aufwärmt. Zum Reaktionsgemisch wurde anschließend bei 0 °C eine Lösung von *N,N-*Dimethylethylendiamin (0.55 mL, 440 mg, 5 mmol) und Triethylamin (0.5 mL) in absolutem Diethylether (30 mL) langsam zugetropft. Das Reaktionsgemisch wurde bei Raumtemperatur 16 h gerührt, danach mit Chlorwasserstoff in Diethylether angesäuert und 1 h bei Raumtemperatur gerührt. Der entstandene Feststoff wurde abgesaugt, mit Diethylether gewaschen und in Vak. getrocknet. Der weiße Feststoff wurde in Wasser (100 mL) suspendiert. Die Suspension auf zwei Zentrifugengläser verteilt, 2 x 20 min bei 5000 U/min zentrifugiert und danach dekantiert. Der isolierte Feststoff **(OG-313.1)** wurde i. Vak. über Phosphorpentoxid getrocknet.
Ausbeute: 206 mg (7 %), weißer schmieriger Feststoff

### OG-313.1

¹H-NMR (DMSO-d₆): 0.80-0.85 (3 H, m); 1.24-1.40 (26 H, m); 1.40-1.46 (2 H, m); 1.72-1.96 (4 H, m); 2.79 (6 H, s); 3.00-3.45 (6 H, m); 3.50-3.62 (2 H, m);3.70-3.80 (2 H, m); 4.00 (1 H, d, *J* = 10.8 Hz); 4.05 (1 H, d, *J* = 11.7 Hz); 7.90-8.10 (1 H, m); 10.13 (1 H, s); 10.40 (1 H, br s).
¹³C-NMR (DMSO-d₆):13.92; 22.06; 25.40; 25.56; 28.65; 28.75; 28.76; 28.98; 30.06; 31.24; 34.43; 42.26 (2 C); 55.75; 66.60; 67.70; 70.72; 72.31; 82.37; 152.88; 153.73.
Im aliphatischen Bereich sind Signale überlagert.

### 4-(N,N,N-Trimethylammonioethyl)allophansäure-(2-hexadecyloxymethyl tetrahydrofuran-2-yl)methylester-lodid (OG-316.1)

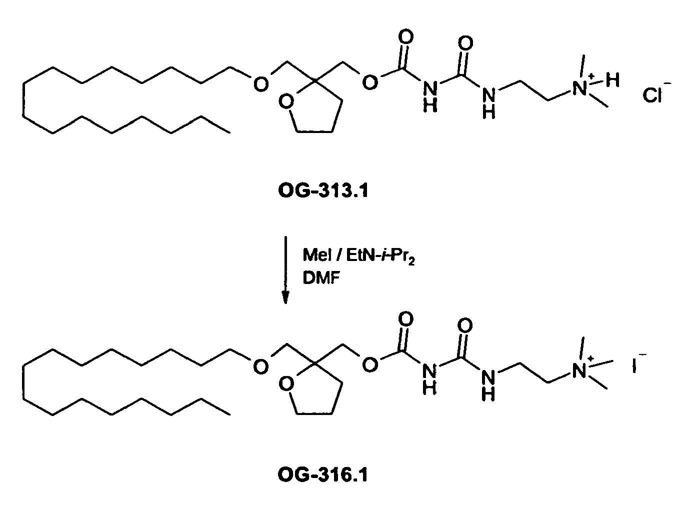

Eine Mischung von **OG-313.1** (170 mg, 0.31 mmol) in absolutem *N,N*-Dimethylformamid (10 mL) und Ethyldiisopropylamin (105 µL, 80 mg, 0.62 mmol) wurde mit Methyliodid (96 µL, 220 mg, 1.55 mmol) versetzt und danach 2.5 h bei 50 °C gerührt. Das Reaktionsgemisch wurde i. Vak. zur Trockne eingeengt, der Rückstand in Dichlormethan aufgenommen, die resultierende Suspension erneut eingeengt und der Feststoff i. Vak. getrocknet. Der Rückstand wurde mit Wasser (30 mL) versetzt und bei Raumtemperatur 30 min gerührt. Die Suspension wurde zentrifugiert (20 min bei 5000 U/min) und danach dekantiert. Der isolierte amorphe Feststoff wurde mit Methanol versetzt, die Suspension eingeengt und der Rückstand anschließend i. Vak. über Phosphorpentoxid getrocknet.
Ausbeute:120 mg (58 %), weißer Feststoff
Schmelzpunkt: 79-85 °C
¹H-NMR (DMSO-d₆): 0.86 (3 H, t, *J* = 6,7 Hz); 1.15-1.36 (26 H, m); 1.45-1.53 (2 H, m); 1.66-1.95 (4 H, m); 3.10 (9 H, s); 3.20-3.48 (6 H, m); 3.54-3.64 (2 H, m); 3.66-3.80 (2 H, m); 3.99 (1 H, d, *J* = 10.8 Hz); 4.05 (1 H, d, *J* = 11.7 Hz); 8.07 (1 H, br t, *J* = 6.3 Hz); 10.21 (1 H, s).
¹³C-NMR (DMSO-d₆): 13.93; 22.04; 25,38; 25.54; 28.63; 28.72; 28.97; 30.08; 31.24; 33.74; 52.47; 63.99; 66.65; 67.69; 70.70; 72.25; 82.37; 152.76; 153.91.
Im aliphatischen Bereich sind Signale überlagert.

### Beispiel 8:

### Stufe 1:

### 4-(Dimethylamino-1-ethyl)allophansäurephenylester (US-605)

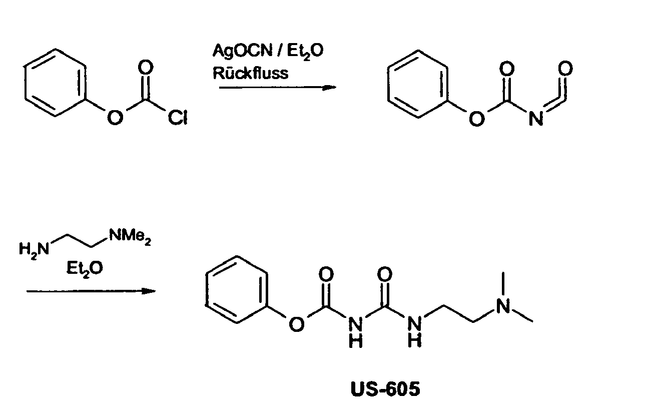

Eine Suspension von Silbercyanat (890 mg, 6 mmol) in absolutem Diethylether (20 mL) wurde tropfenweise mit einer Lösung von Chlorameisensäurephenylester (780 mg, 630 µL, 5 mmol) in absolutem Diethylether (20 mL) versetzt und anschließend 1 h unter Rückfluss gerührt. Nach dem Abkühlen wurde der ausgefallene Feststoff abfiltriert. Das Filtrat wurde zu einer Lösung von *N¹,N¹*-Dimethylethan-1,2-diamin (530 mg, 660 µL, 6 mmol) in Diethylether (25 mL) getropft, dabei fiel ein weißer Feststoff (427 mg) aus, der abfiltriert wurde. Das Filtrat wurde i. Vak. eingeengt (660 mg). Der Rückstand wurde durch Flashchromatographie (18 g, 20 x 2.0 cm) mit Ethylacetat / Cyclohexan (1:1) und 1 % Triethylamin gereinigt.
Ausbeute: 230 mg (18 %), gelbes Öl
¹H-NMR (DMSO-d₆): 2.16 (6 H, s); 2.34 (2 H, t, *J* = 6.8 Hz); 3.14 (2 H, dd, *J* = 12.0 und 6.0 Hz); 6.75 (1 H, m); 7.06 (2 H, d, *J* = 7.8 Hz); 7.10-7.21 (1 H, m); 7.36 (2 H, t, *J* = 7.8 Hz); 7.60 (1 H, br t, *J* = 5.3 Hz).
¹³C-NMR (DMSO-d₆): 36.6; 45.2 (2 C) ; 58.2; 121.6 (2 C); 124.7; 129.3 (2 C); 151.1; 154.2; 157.3.

### Stufe 2:

### 4-(Trimethylammonio-ethyl)allophansäurephenylester-Iodid (US-608)

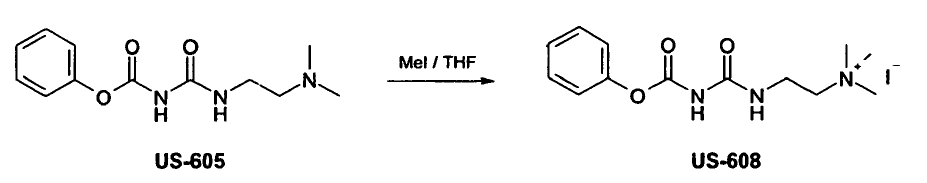

Eine Lösung von US-605 (130 mg, 0.52 mmol) in absolutem Tetrahydrofuran (20 mL) wurde mit Methyliodid (355 mg, 150 µL, 2.5 mmol) versetzt und bei Raumtemperatur 4 h gerührt. Der ausgefallene Niederschlag wurde abfiltriert und i. Vak. getrocknet. Ausbeute: 100 mg (49 %), weißer, hygroskopischer Schaum
Schmelzpunkt: 30-125 °C
¹H-NMR (DMSO-d₆, nach 5 Tagen): 3.11 (9 H, s); 3.34 (2 H, t, *J* = 6.3 Hz); 3.44 (2 H, m); 6.67 (1 H, t, *J* = 5.5 Hz); 6.74 (1 H, s); 6.75 (2 H, m); 7.15 (2 H, dd, *J* = 8.6 und 8.6 Hz); 9.33 (1 H, br s).
¹³C-NMR (DMSO-d₆): 33.8; 52.6 (3 C); 64.6 (2 C); 115.1 (2 C); 118.7; 129.3 (3 C); 1.54.4; 157.2; 157.7.
Die Substanz ist ein Tautomerengemisch und enthält noch Tetrahydrofuran.

### Beispiel 9:

### Stufe 1:

### Chlorameisensäurebiphenyl-4-yl-ester (US-617)

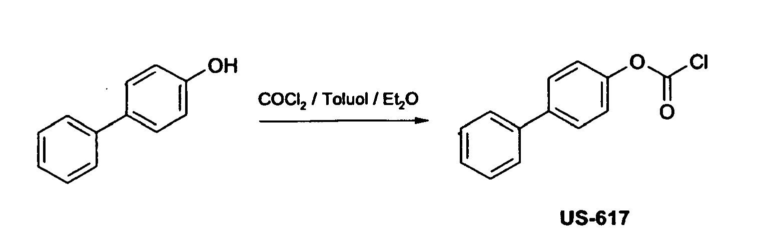

Zu einer Lösung von Phosgen (1.98 g, 20 mmol) in absoluten Toluol (20 mL) wurde unter Eiskühlung 4-Hydroxybiphenyl (2.62 g, 15.4 mmol) in absolutem Toluol /Diethylether (60 / 30 mL) innerhalb 30 Minuten getropft. Anschließend wurde bei -3 °C *N,N*-Dimethylanilin (1.86 g, 1.95 mL, 15.4 mmol) in absolutem Toluol (10 mL) zugetropft und über Nacht unter langsamer Erwärmung auf Raumtemperatur gerührt. Nach erneuter Kühlung auf 0 °C wurde langsam Wasser (30 mL) zugetropft, anschließend wurde die organische Phase mit 0.1 *N* HCl, 0.1 *N* NaOH und Wasser (je 2x 100 mL) gewaschen, mit Natriumsulfat getrocknet und i. Vak. eingeengt. Ausbeute: 2.69 g (75 %), farbloses Öl
¹H-NMR (DMSO-d₆): 6.88 (2 H, d, *J* = 7.6 Hz); 7.26 (1 H, t, *J* = 7.2 Hz); 7.39 (2 H, t, *J* = 7.2 Hz); 7.47 (2 H, d, *J* = 8.4 Hz); 7.56 (2 H, d, *J* = 7.6 Hz).
¹³C-NMR (DMSO-d₆): 115.7; 125.9; 126.3; 127.7; 128.8; 128.9; 130.9; 140.2; 157.1.

### Stufe 2:

### 4-(Dimethylamino-1-ethyl)allophansäure-(4-phenyl)-phenylester (US-620)

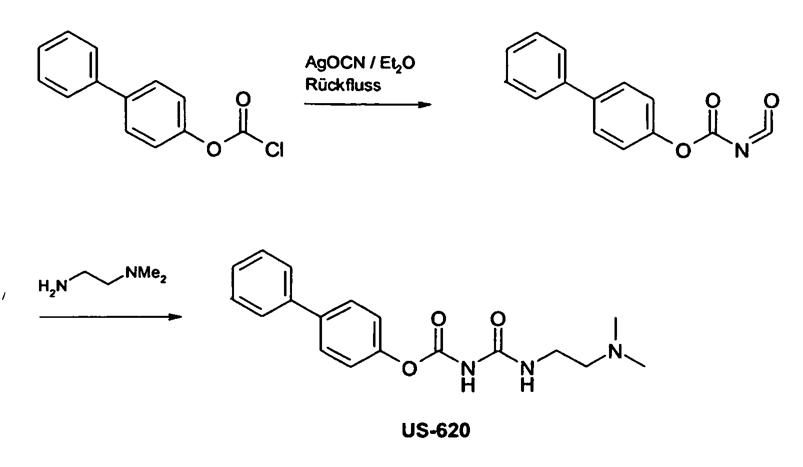

Eine Suspension von Silbercyanat (2.10 g, 13.8 mmol) in absolutem Diethylether (20 mL) wurde tropfenweise mit einer Lösung von **US-617** (2.70 g, 11.5 mmol) in absolutem Diethylether (20 mL) versetzt und anschließend 1 h unter Rückfluss gerührt. Nach dem Abkühlen wurde der ausgefallene Feststoff abfiltriert. Das Filtrat wurde zu einer Lösung von *N¹,N¹*-Dimethylethan-1,2-diamin (1.20 g, 1.5 mL, 13.8 mmol) in Diethylether (25 mL) getropft, dabei fiel ein weißer Feststoff aus, der abfiltriert wurde. Das Filtrat wurde i. Vak. eingeengt. Durch Flashchromatographie konnte die Verbindung nicht gereinigt werden, da sie sich auf der Säule zersetzt. Rohausbeute: 2.60 g (70 %), gelblicher, kristalliner Feststoff
¹H-NMR (DMSO-d₆): 2.17 (6 H, s); 2.33 (2 H, m); 3.16 (2 H, m); 6.86 (1 H, d, *J*=
8.8 Hz); 7.10-7.80 (9 H, m); 9.57 (1 H, s). Daneben existieren weitere Signale, die nicht zur Verbindung gehören.
¹H-NMR (DMSO-d₆): 1 C unter DMSO; 45.1; 58.2; 115.6; 121.6; 122.1; 125.2; 125.8; 126.5; 127.4; 127.6; 128.1; 128.7; 150.6; 154.2; 157.0.

### Stufe 3:

### 4-(Trimethylammonio-1-ethyl)allophansäure-(4-phenyl)-phenylester-Iodid (US-624)

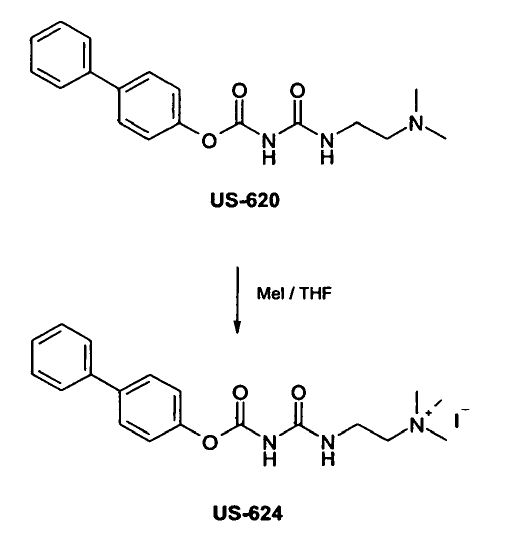

Eine Lösung von rohem **US-620** (280 mg, 0.85 mmol) in absolutem Tetrahydrofuran (20 mL) wurde mit Methyliodid (560 mg, 240 µL, 4 mmol) versetzt und bei Raumtemperatur 5 h gerührt. Der ausgefallene Niederschlag wurde abfiltriert und getrocknet.
Ausbeute: 247 mg (62 %), weißer Feststoff
Schmelzpunkt: 95-125 °C
¹H-NMR (DMSO-d₆, nach 3 Tagen): 3.10 (9 H; s); 3.34 (2 H, t, *J* = 6.6 Hz); 3.44 (2 H, br m); 6.66 (1 H, br t); 6.84 (2 H, d, *J* = 8.6 Hz); 7.27 (1 H, t, *J* = 7.0 Hz); 7.40 (2 H, t, *J* = 7.8 Hz); 7.47 (2 H, d, *J* = 8.6 Hz); 7.60 (2 H, d, *J* = 7.0 Hz), 9.55 (1 H, br s).
¹³C-NMR (DMSO-d₆, nach 3 Tagen): 33.8; 52.6; 64.6; 115.7; 125.9; 126.3; 127.6; 128.7; 130.9; 140.2; 148.5; 157.1; 157.8.

Sofern in einer der vorstehenden Umsetzungen eine Verbindung mit zwei unterschiedlichen Bezeichnungen versehen ist, beispielsweise RK324 und IS327, handelt es sich unterschiedliche Fraktionen ein- und derselben Verbindung.

### Pharmakologische Daten:

Die untersuchten erfindungsgemäßen Salze zeigen eine ausgezeichnete Affinität zu dem Vanilloid-Rezeptor 1 (VR1/TRPV1-Rezeptor). Die folgende Tabelle gibt die gemäß obenstehender Methode II erhaltenen Bestimmungen wieder:

| | hVR1 10 µM Inhibierung | rVR1 10 µM Stimulierung | rVR1 10 µM Inhibierung | IC50 µM hVR1 | IC50 µM rVR1 |
|---|---|---|---|---|---|
| 1 | 95,44 | 0,55 | 103,15 | 2,43 | 2,1 |
| 2 | 88,93 | -0,02 | 87,13 | 0,92 | 1,23 |
| 3 | 56,92 | -0,73 | 94,41 | 4,27 | 1,27 |
| 13 | 80,84 | 0,36 | 92,11 | 1,1 | 1,3 |
| 14 | 93,08 | 0,09 | 95,54 | 2,4 | 1,15 |
| 15 | 101,37 | -1,01 | 108,94 | 3,45 | 2,95 |
| 16 | 68,63 | 0,58 | 99,30 | 1,7 | 2,9 |

## Patentansprüche

1. Salze substituierter Allophansäureester aus einem kationischen Salzpartner der allgemeinen Formel 1, 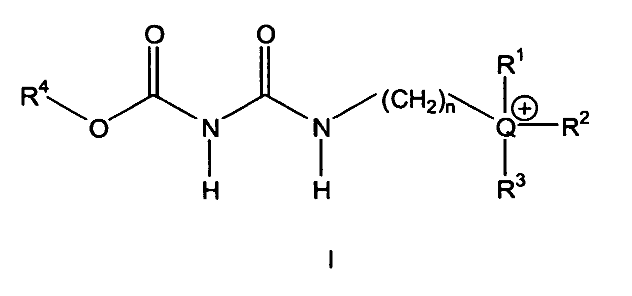 worin
n = 1, 2, 3, 4, 5 oder 6 ist;
Q für N oder P steht;
R¹, R², R³, unabhängig voneinander, jeweils für einen linearen oder verzweigten C₁₋₅-Alkyl-Rest stehen;
oder zwei dieser Reste zusammen mit dem sie verbindenden Atom Q als Ringglied einen 4-, 5-, 6- oder 7-gliedrigen, gesättigten oder ungesättigten, unsubstituierten oder einfach oder mehrfach substituierten, ggf. wenigstens ein weiteres Heteroatom als Ringglied aufweisenden Heterozyklus bilden und jeweils der verbleibende Rest die vorstehend genannte Bedeutung hat;
R⁴ für einen linearen oder verzweigten, gesättigten oder ungesättigten, unsubstituierten oder einfach oder mehrfach substituierten, ggf. wenigstens ein Heteroatom als Kettenglied aufweisenden aliphatischen Rest,
für einen ungesättigten oder gesättigten, unsubstituierten oder einfach oder mehrfach substituierten, ggf. wenigstens ein Heteroatom als Ringglied aufweisenden cycloaliphatischen Rest, der über eine lineare oder verzweigte Alkylen-Gruppe gebunden sein kann,
für einen unsubstituierten oder wenigstens einfach oder mehrfach substituierten, ggf. über eine lineare oder verzweigte Alkylen-Gruppe gebundenen Aryl-Rest oder Heteroaryl-Rest steht, oder
für eine Gruppe -(CH₂)_{q}-Aᵣ-(CH₂)ₛ-B-(CH₂)ₜ-Cᵤ-R⁵ steht, in der
q, s und t, unabhängig voneinander, jeweils für 0, 1, 2, 3, 4, 5 oder 6 stehen;
r und u, unabhängig voneinander, jeweils für 0 oder 1 stehen;
A und C, unabhängig voneinander, jeweils für O, S oder NH stehen;
B für eine lineare oder verzweigte, unsubstituierte oder einfach oder mehrfach substituierte Alkylen-, Alkenylen- oder Alkinylen-Gruppe steht; für einen ungesättigten oder gesättigten, unsubstituierten oder einfach oder mehrfach substituierten, ggf. wenigstens ein Heteroatom als Ringglied aufweisenden cycloaliphatischen Rest; oder für einen unsubstituierten oder einfach oder mehrfach substituierten Aryl-Rest oder Heteroaryl-Rest steht; und
R⁵ für einen linearen oder verzweigten, gesättigten oder ungesättigten, unsubstituierten oder einfach oder mehrfach substituierten aliphatischen Rest steht,
jeweils ggf. in Form eines seiner reinen Stereoisomeren, insbesondere Enantiomeren oder Diastereomeren, seines Racemates oder in Form einer Mischung von Stereoisomeren, insbesondere der Enantiomeren und/oder Diastereomeren, in einem beliebigen Mischungsverhältnis,
und einem anionischen Salzpartner.

2. Salze gemäß Anspruch 1, **dadurch gekennzeichnet, dass**
R¹, R², R³, unabhängig voneinander, jeweils für einen Alkyl-Rest stehen, der ausgewählt ist aus der Gruppe bestehend aus Methyl, Ethyl, n-Propyl und iso-Propyl,
oder zwei dieser Reste zusammen mit dem sie verbindenden Atom Q als Ringglied einen 4-, 5-, 6- oder 7-gliedrigen, gesättigten oder ungesättigten, unsubstituierten oder einfach oder mehrfach substituierten, ggf. wenigstens ein weiteres Heteroatom als Ringglied aufweisenden Heterozyklus bilden und jeweils der verbleibende Rest für einen Alkyl-Rest steht, der ausgewählt ist aus der Gruppe bestehend aus Methyl, Ethyl, n-Propyl und iso-Propyl.

3. Salze gemäß Anspruch 1 oder 2, **dadurch gekennzeichnet, dass**
R⁴ für einen linearen oder verzweigten, gesättigten oder ungesättigten, unsubstituierten oder einfach oder mehrfach substituierten aliphatischen C₁₋₃₀ Rest;
für einen ungesättigten oder gesättigten, unsubstituierten oder einfach oder mehrfach substituierten, ggf. ein, zwei oder drei Heteroatome als Ringglied aufweisenden, 5-, 6- oder 7-gliedrigen cycloaliphatischen Rest, der über eine lineare oder verzweigte C₁₋₃ Alkylen-Gruppe gebunden sein kann,
für einen unsubstituierten oder wenigstens einfach substituierten, ggf. über eine lineare oder verzweigte C₁₋₃-Alkylen-Gruppe gebundenen 5- oder 6-gliedrigen Aryl-Rest oder Heteroaryl-Rest steht, oder
für eine Gruppe -(CH₂)_{q}-Aᵣ-(CH₂)ₛ-B-(CH₂)ₜ-Cᵤ-R⁵ steht, in der
q, s und t, unabhängig voneinander, jeweils für 0, 1, 2, 3, 4, 5 oder 6 stehen;
r und u, unabhängig voneinander, jeweils für 0 oder 1 stehen;
A und C, unabhängig voneinander, jeweils für O oder S stehen;
B für eine lineare oder verzweigte C₁₋₆-Alkylen-, C₂₋₆-Alkenylen- oder C₂₋₆-Alkinylen-Gruppe steht; die mit 1, 2 oder 3 Substituenten unabhängig voneinander ausgewählt aus der Gruppe bestehend aus F, Cl, Br, OH, -O-C₁₋₃-Alkyl, SH und -S-C₁₋₃-Alkyl substituiert sein kann,
für einen (hetero)cycloaliphatischen Rest ausgewählt aus der Gruppe bestehend aus Cyclopropyl, Cylobutyl, Cyclopentyl, Cyclohexyl, Cyclohexyl, Cyclooctyl, Cyclononyl, Cyclopentenyl, Cyclohexenyl, Cycloheptenyl, Cyclooctenyl, Imidazolinyl, Aziridinyl, Azetidinyl, Pyrrolidinyl, Piperidinyl, Azepanyl, Azokanyl, Piperazinyl, Tetrahydrofuranyl (Tetrahydrofuryl), Tetrahydrothienyl (Tetrahydrothiophenyl), Morpholinyl und Thiomorpholinyl,
oder für einen (Hetero)aryl-Rest ausgewählt aus der Gruppe bestehend aus Pyrrolyl, Indolyl, Furyl (Furanyl), Benzo[b]furanyl, Thienyl (Thiophenyl), Benzo[b]thienyl, Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl und Chinazolinyl steht, und
R⁵ für einen linearen oder verzweigten, gesättigten oder ungesättigten, unsubstituierten oder einfach oder mehrfach substituierten aliphatischen C₁₋₃₀-Rest steht.

4. Salze gemäß einem oder mehreren der Ansprüche 1-3, **dadurch gekennzeichnet, dass**
n = 1, 2, 3, 4, 5 oder 6 ist;
Q für N oder P steht;
R¹, R², R³, unabhängig voneinander, jeweils für einen Alkyl-Rest stehen, der ausgewählt ist aus der Gruppe bestehend aus Methyl, Ethyl, n-Propyl und iso-Propyl,
oder zwei dieser Reste zusammen mit dem sie verbindenden Atom Q als Ringglied einen 4-, 5-, 6- oder 7-gliedrigen, gesättigten, unsubstituierten Heterozyklus bilden und jeweils der verbleibende Rest für einen Alkyl-Rest steht, der ausgewählt ist aus der Gruppe bestehend aus Methyl, Ethyl, n-Propyl und iso-Propyl,
R⁴ für einen linearen oder verzweigten, gesättigten oder ungesättigten aliphatischen C₁₋₂₀ Rest;
für einen ggf. über eine C₁₋₃-Alkylen-Gruppe gebundenen Aryl- oder Heteroaryl-Rest ausgewählt aus der Gruppe bestehend aus Phenyl, Naphthyl, Furanyl, Thiophenyl und Pyridinyl, wobei der zyklische Teil dieser Reste jeweils unsubstituiert oder mit 1, 2, 3, 4 oder 5 Substituenten ausgewählt aus der Gruppe bestehend aus F, Cl, Br, linearem oder verzweigtem C₁₋₃-Alkyl, linearem oder verzweigtem C₁₋₃-Alkoxy, Phenyl, Phenoxy, Benzyl und Benzyloxy substituiert sein kann;
für eine Gruppe -(CH₂)_{q}-Ar(CH₂)ₛ-B-(CH₂)ₜ-Cᵤ-R⁵ steht, in der
q, s und t, unabhängig voneinander, jeweils für 0, 1 oder 2 stehen;
r und u, unabhängig voneinander, jeweils für 0 oder 1 stehen;
A und C, unabhängig voneinander, jeweils für O stehen;
B für eine lineare oder verzweigte C₁₋₆-Alkylen-, C₂₋₆-Alkenylen- oder C₂₋₆-Alkinylen-Gruppe steht; die mit 1, 2 oder 3 Substituenten unabhängig voneinander ausgewählt aus der Gruppe bestehend aus F, Cl, Br, OH, -O-C₁₋₃-Alkyl, SH und -S-C₁₋₃-Alkyl substituiert sein kann,
für einen (hetero)cycloaliphatischen Rest ausgewählt aus der Gruppe bestehend aus Cyclopropyl, Cylobutyl, Cyclopentyl, Cyclohexyl, Cyclohexyl, Cyclooctyl, Cyclononyl, Cyclopentenyl, Cyclohexenyl, Cycloheptenyl, Cyclooctenyl, Imidazolinyl, Aziridinyl, Azetidinyl, Pyrrolidinyl, Piperidinyl, Azepanyl, Azokanyl, Piperazinyl, Tetrahydrofuranyl (Tetrahydrofuryl), Tetrahydrothienyl (Tetrahydrothiophenyl), Morpholinyl und Thiomorpholinyl,
oder für einen (Hetero)aryl-Rest ausgewählt aus der Gruppe bestehend aus Pyrrolyl, Indolyl, Furyl (Furanyl), Benzo[b]furanyl, Thienyl (Thiophenyl), Benzo[b]thienyl, Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl und Chinazolinyl steht, und
R⁵ für einen linearen oder verzweigten, gesättigten oder ungesättigten aliphatischen Rest C₁₋₂₀-Rest steht.

5. Salze gemäß einem oder mehreren der Ansprüche 1-4 auf Basis eines kationischen Salzpartners der allgemeinen Formel la 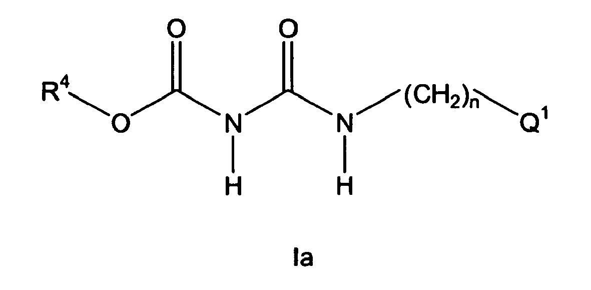 worin
n für 1, 2 oder 3 steht;
Q¹ für einen Rest steht, der ausgewählt ist aus der Gruppe bestehend aus 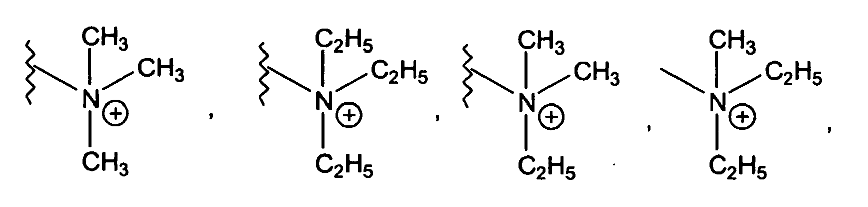 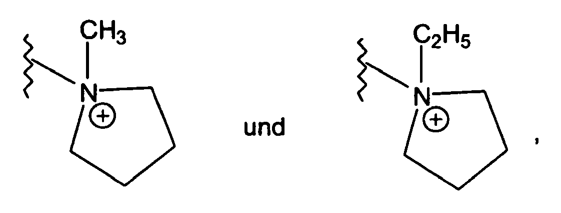 R⁴ für einen linearen oder verzweigten C₁₋₂₀ Alkyl-Rest;
für einen Phenyl- oder Benzyl-Rest, wobei der zyklische Teil dieser Reste unsubstituiert oder mit 1, 2, 3, 4 oder 5 Substituenten ausgewählt aus der Gruppe bestehend aus F, Cl, Br, linearem oder verzweigtem C₁₋₃-Alkyl, linearem oder verzweigtem C₁₋₃-Alkoxy, Phenyl, Phenoxy, Benzyl und Benzyloxy substituiert sein kann;
oder für einen Rest ausgewählt aus der Gruppe bestehend aus 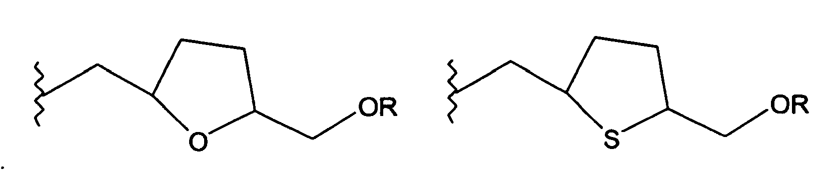 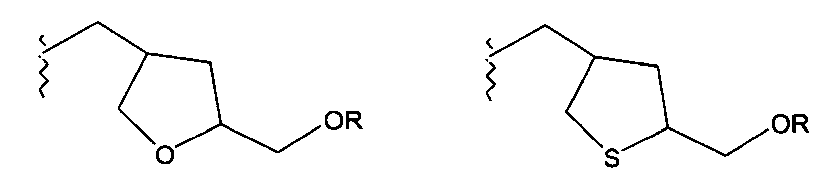 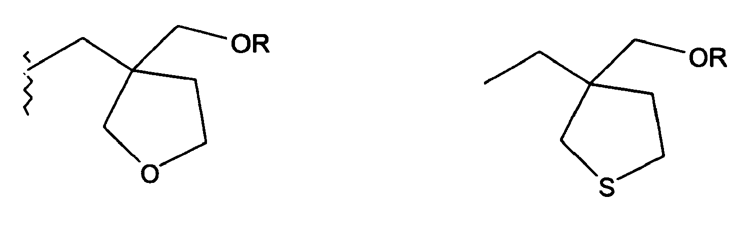 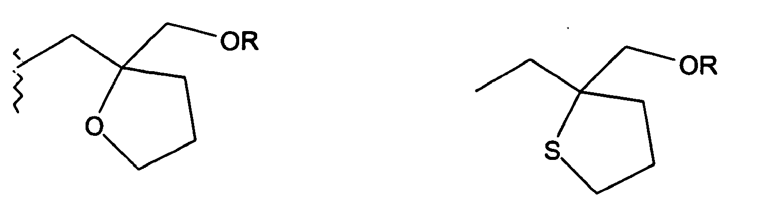 worin R jeweils für einen linearen oder verzweigten C₁₋₂₀-Alkyl-Rest steht.

6. Salze gemäß Anspruch 5, **dadurch gekennzeichnet, dass**
n für 1, 2 oder 3 steht,
Q¹ für einen Rest steht, der ausgewählt ist aus der Gruppe bestehend aus 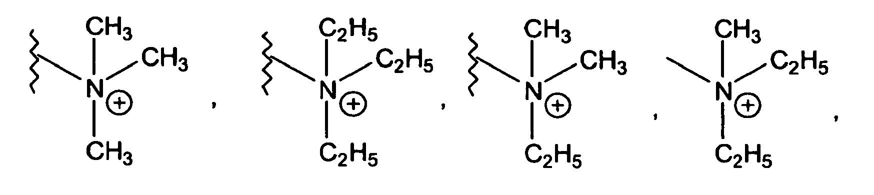 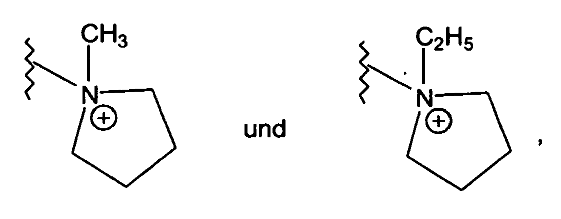 R⁴ für einen Alkyl-Rest ausgewählt aus der Gruppe bestehend aus Methyl, Ethyl, n-Propyl, n-Butyl, n-Pentyl, n-Hexyl, n-Heptyl, n-Octyl, n-Nonyl, n-Decanyl, n-Undecanyl, n-Dodecanyl, n-Tridecanyl, n-Tetradecanyl, n-Pentadecanyl, n-Hexadecanyl, n-Heptadecanyl, n-Octadecanyl, n-Nonadecanyl und n-Eicosanyl;
für einen Phenyl- oder Benzyl-Rest, wobei der zyklische Teil dieser Reste unsubstituiert oder einfach mit einem Substituenten ausgewählt aus der Gruppe bestehend aus F, Cl, Br, linearem oder verzweigtem C₁₋₃-Alkyl, linearem oder verzweigtem C₁₋₃-Alkoxy, Phenyl, Phenoxy, Benzyl und Benzyloxy substituiert sein kann;
oder für einen der folgenden Reste steht, 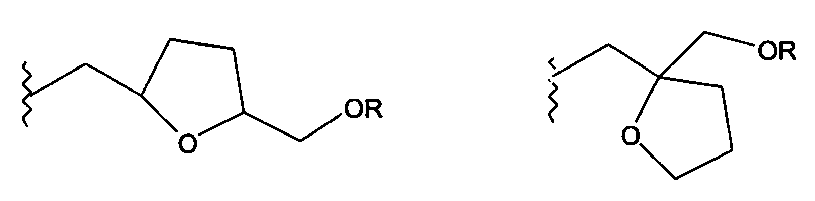 worin R jeweils für einen Alkyl-Rest ausgewählt aus der Gruppe bestehend aus Methyl, Ethyl, n-Propyl, n-Butyl, n-Pentyl, n-Hexyl, n-Heptyl, n-Octyl, n-Nonyl, n-Decanyl, n-Undecanyl, n-Dodecanyl, n-Tridecanyl, n-Tetradecanyl, n-Pentadecanyl, n-Hexadecanyl, n-Heptadecanyl, n-Octadecanyl, n-Nonadecanyl und n-Eicosanyl steht.

7. Salze gemäß einem oder mehreren der Ansprüche 1-6, **dadurch gekennzeichnet, dass** der anionische Salzpartner ein Halogenid-Ion oder ein Hydroxyl-lon ist, vorzugsweise ein Chlorid-, Bromid- oder lodid-lon, besonders bevorzugt ein Iodid-Ion ist.

8. Salze substituierter Allophansäureester gemäß einem oder mehreren der Ansprüche 1-7 ausgewählt aus der Gruppe bestehend aus
[1] 4-(Trimethylamino-1-ethyl)allophansäure-dodecylester-iodid,
[2] 4-(Pyrrolidinium-1-ethyl)allophansäure-hexadecylester-iodid,
[3] 4-(Trimethylamino-1-propyl)allophansäure-hexadecylester-iodid,
[4] 4-(Trimethylamino-1-ethyl)allophansäure-octylester-iodid,
[5] 4-(Trimethylamino-1-ethyl)allophansäure-butylester-iodid,
[6] 4-(Trimethylamino-1-ethyl)allophansäure-phenylester-iodid,
[7] 4-Benzyloxycarbonyl-4-(N-methyldimethylammonium-1-ethyl)allophansäure-benzylester-iodid,
[8] 4-(Trimethylamino-1-ethyl)allophansäure-benzylester-iodid,
[9] 4-(Trimethylamino-1-ethyl)allophansäure-(4-phenyl)-phenylester-iodid,
[10] 4-(Trimethylamino-1-ethyl)allophansäure-ethylester-iodid,
[11] 4-(Trimethylamino-1-ethyl)allophansäure-butylester-iodid,
[12] 4-(Trimethylamino-1-ethyl)allophansäure-hexylester-iodid,
[13] 4-(Trimethylamino-1-ethyl)allophansäure-(cis-5-hexadecyloxymethyltetrahydrofuran-2-yl)methylester-iodid,
[14] 4-(Trimethylamino-1-ethyl)allophansäure-3-hexadecyloxy-2-methoxypropan-1-yl-ester-iodid,
[15] 4-(Trimethylamino-1-ethyl)allophansäure-hexadecylester-iodid und
[16] 4-(Trimethylamino-1-ethyl)allophansäure-(2-hexadecyloxymethyltetrahydrofuran-2-yl)methylester-iodid.

9. Verfahren zur Herstellung substituierter Allophansäureester gemäß einem oder mehreren der Ansprüche 1-8, **dadurch gekennzeichnet, dass** man
eine Verbindung der allgemeinen Formel II, 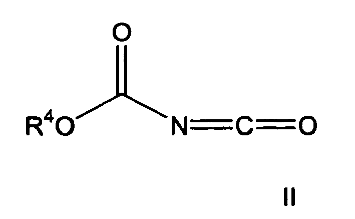 worin R⁴ die Bedeutung gemäß einem oder mehreren der Ansprüche 1-8 hat, in einem Reaktionsmedium, ggf. in Gegenwart einer Base, mit einer Verbindung der allgemeinen Formel III 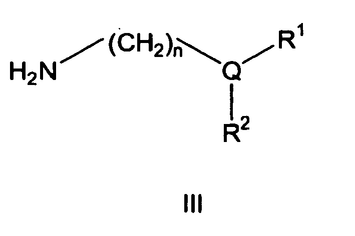 worin n, R¹ und R² die Bedeutung gemäß einem oder mehreren der Ansprüche 1-8 hat, zu einer Verbindung der allgemeinen Formel IV umsetzt, 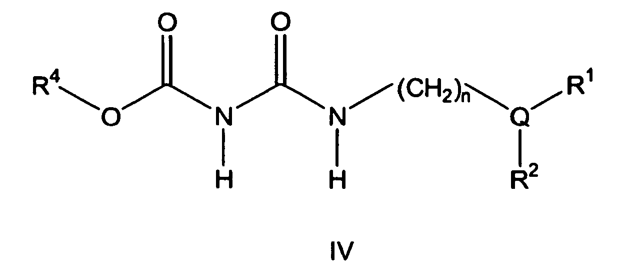 worin R¹, R², R⁴ und n die vorstehend genannte Bedeutung haben, und die so erhaltene Verbindung der allgemeinen Formel IV durch Umsetzung mit einem Alkylierungsmittel in eine Verbindung der allgemeinen Formel überführt.

10. Arzneimittel enthaltend wenigstens ein Salz gemäß einem oder mehreren der Ansprüche 1 bis 8 und ggf. einen oder mehrere physiologisch verträgliche Hilfsstoffe.

11. Arzneimittel gemäß Anspruch 10 zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

12. Verwendung wenigstens eines Salzes gemäß einem oder mehreren der Ansprüche 1 bis 8 zur Herstellung eines Arzneimittels zur Behandlung und/oder Prophylaxe von einer oder mehreren Erkrankungen ausgewählt aus der Gruppe bestehend Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen;; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

13. Verwendung wenigstens eines Salzes gemäß einem oder mehreren der Ansprüche 1 bis 8 zur Herstellung eines Arzneimittels zur Behandlung und/oder Prophylaxe von Schmerz ausgewählt aus der Gruppe bestehend aus akutem Schmerz, chronischem Schmerz, neuropathischem Schmerz und visceralem Schmerz.

## Claims

1. Salts of substituted allophanates from a cationic salt partner of general formula I, 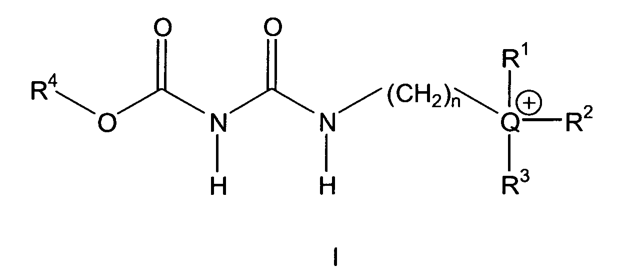 in which
n = 1, 2, 3, 4, 5 or 6;
Q represents N or P;
R¹, R², R³, independently of one another, each represent a linear or branched C₁₋₅ alkyl residue;
or two of these residues, together with the atom Q joining them as ring member, form a 4-, 5-, 6- or 7-membered, saturated or unsaturated heterocycle, unsubstituted or substituted one or more times, optionally having at least one further heteroatom as ring member and in each case the remaining residue has the meaning stated previously;
R⁴ represents a linear or branched, saturated or unsaturated aliphatic residue, unsubstituted or substituted one or more times, optionally having at least one heteroatom as a unit of the chain,
an unsaturated or saturated cycloaliphatic residue, unsubstituted or substituted one or more times, optionally having at least one heteroatom as ring member, which can be joined via a linear or branched alkylene group,
an aryl residue or heteroaryl residue, unsubstituted or substituted at least one or more times, optionally joined via a linear or branched alkylene group, or
represents a group -(CH₂)_{q}-Aᵣ-(CH₂)ₛ-B-(CH₂)ₜ-Cᵤ-R⁵, in which
q, s and t, independently of one another, each represent 0, 1, 2, 3, 4, 5 or 6;
r and u, independently of one another, each represent 0 or 1;
A and C, independently of one another, each represent O, S or NH;
B represents a linear or branched alkylene, alkenylene or alkynylene group, unsubstituted or substituted one or more times; an unsaturated or saturated cycloaliphatic residue, unsubstituted or substituted one or more times, optionally having at least one heteroatom as ring member; or an aryl residue or heteroaryl residue, unsubstituted or substituted one or more times; and
R⁵ represents a linear or branched, saturated or unsaturated aliphatic residue, unsubstituted or substituted one or more times,
in each case optionally in the form of one of its pure stereoisomers, in particular enantiomers or diastereomers, its racemate or in the form of a mixture of stereoisomers, in particular of the enantiomers and/or diastereomers, in any proportions,
and an anionic salt partner.

2. Salts according to claim 1, **characterised in that**
R¹, R², R³, independently of one another, each represent an alkyl residue, which is selected from the group consisting of methyl, ethyl, n-propyl and isopropyl,
or two of these residues, together with the atom Q joining them as ring member, from a 4-, 5-, 6- or 7-membered, saturated or unsaturated heterocycle, unsubstituted or substituted one or more times, optionally having at least one further heteroatom as ring member, and in each case the remaining residue represents an alkyl residue, which is selected from the group consisting of methyl, ethyl, n-propyl and isopropyl.

3. Salts according to claim 1 or 2, **characterised in that**
R⁴ represents a linear or branched, saturated or unsaturated aliphatic C₁₋₃₀ residue, unsubstituted or substituted one or more times;
an unsaturated or saturated 5-, 6- or 7-membered cycloaliphatic residue, unsubstituted or substituted one or more times, optionally having one, two or three heteroatoms as ring member, which can be bound via a linear or branched C₁₋₃ alkylene group,
a 5- or 6-membered aryl residue or heteroaryl residue, unsubstituted or substituted at least once, optionally bound via a linear or branched C₁₋₃ alkylene group, or
represents a group -(CH₂)_{q}-Aᵣ-(CH₂)ₛ-B-(CH₂)ₜ-Cᵤ-R⁵, in which
q, s and t, independently of one another, each represent 0, 1, 2, 3, 4, 5 or 6;
r and u, independently of one another, each represent 0 or 1;
A and C, independently of one another, each represent O or S;
B represents a linear or branched C₁₋₆ alkylene, C₂₋₆ alkenylene or C₂₋₆ alkynylene group; which can be substituted with 1, 2 or 3 substituents selected independently of one another from the group consisting of F, Cl, Br, OH, -O-C₁₋₃-alkyl, SH and -S-C₁₋₃-alkyl,
a (hetero)cycloaliphatic residue selected from the group consisting of cyclopropyl, cyclobutyl, cyclopentyl, cyclohexyl, cyclohexyl, cyclooctyl, cyclononyl, cyclopentenyl, cyclohexenyl, cycloheptenyl, cyclooctenyl, imidazolinyl, aziridinyl, azetidinyl, pyrrolidinyl, piperidinyl, azepanyl, azocanyl, piperazinyl, tetrahydrofuranyl (tetrahydrofuryl), tetrahydrothienyl (tetrahydrothiophenyl), morpholinyl and thiomorpholinyl,
or represents a (hetero)aryl residue selected from the group consisting of pyrrolyl, indolyl, furyl (furanyl), benzo[b]furanyl, thienyl (thiophenyl), benzo[b]thienyl, pyrazolyl, imidazolyl, thiazolyl, thiadiazolyl, triazolyl, oxazolyl, isoxazolyl, pyridinyl, pyridazinyl, pyrimidinyl, pyrazinyl, pyranyl, indazolyl, purinyl, indolizinyl, quinolinyl, isoquinolinyl and quinazolinyl, and
R⁵ represents a linear or branched, saturated or unsaturated aliphatic C₁₋₃₀ residue, unsubstituted or substituted one or more times.

4. Salts according to one or more of claims 1-3, **characterised in that**
n = 1, 2, 3, 4, 5 or 6;
Q represents N or P;
R¹, R², R³, independently of one another, each represent an alkyl residue, which is selected from the group consisting of methyl, ethyl, n-propyl and isopropyl,
or two of these residues, together with the atom Q joining them as ring member, form a 4-, 5-, 6- or 7-membered, saturated, unsubstituted heterocycle and in each case the remaining residue represents an alkyl residue, which is selected from the group consisting of methyl, ethyl, n-propyl and isopropyl,
R⁴ represents a linear or branched, saturated or unsaturated aliphatic C₁₋₂₀ residue;
an aryl- or heteroaryl residue optionally bound via a C₁₋₃ alkylene group and selected from the group consisting of phenyl, naphthyl, furanyl, thiophenyl and pyridinyl, and the cyclic moiety of these residues can in each case be unsubstituted or substituted with 1, 2, 3, 4 or 5 substituents selected from the group consisting of F, Cl, Br, linear or branched C₁₋₃ alkyl, linear or branched C₁₋₃ alkoxy, phenyl, phenoxy, benzyl and benzyloxy;
represents a group -(CH₂)_{q}-Aᵣ-(CH₂)ₛ-B-(CH₂)ₜ-Cᵤ-R⁵, in which
q, s and t, independently of one another, each represent 0, 1 or 2;
r and u, independently of one another, each represent 0 or 1;
A and C, independently of one another, each represent O;
B represents a linear or branched C₁₋₆ alkylene, C₂₋₆-alkenylene or C₂₋₆-alkynylene group; which can be substituted with 1, 2 or 3 substituents selected independently of one another from the group consisting of F, Cl, Br, OH, -O-C₁₋₃-alkyl, SH and -S-C₁₋₃-alkyl,
represents a (hetero)cycloaliphatic residue selected from the group consisting of cyclopropyl, cyclobutyl, cyclopentyl, cyclohexyl, cyclohexyl, cyclooctyl, cyclononyl, cyclopentenyl, cyclohexenyl, cycloheptenyl, cyclooctenyl, imidazolinyl, aziridinyl, azetidinyl, pyrrolidinyl, piperidinyl, azepanyl, azocanyl, piperazinyl, tetrahydrofuranyl (tetrahydrofuryl), tetrahydrothienyl (tetrahydrothiophenyl), morpholinyl and thiomorpholinyl,
or represents a (hetero)aryl residue selected from the group consisting of pyrrolyl, indolyl, furyl (furanyl), benzo[b]furanyl, thienyl (thiophenyl), benzo[b]thienyl, pyrazolyl, imidazolyl, thiazolyl, thiadiazolyl, triazolyl, oxazolyl, isoxazolyl, pyridinyl, pyridazinyl, pyrimidinyl, pyrazinyl, pyranyl, indazolyl, purinyl, indolizinyl, quinolinyl, isoquinolinyl and quinazolinyl, and
R⁵ represents a linear or branched, saturated or unsaturated C₁₋₂₀ aliphatic residue.

5. Salts according to one or more of claims 1-4 based on a cationic salt partner of general formula la 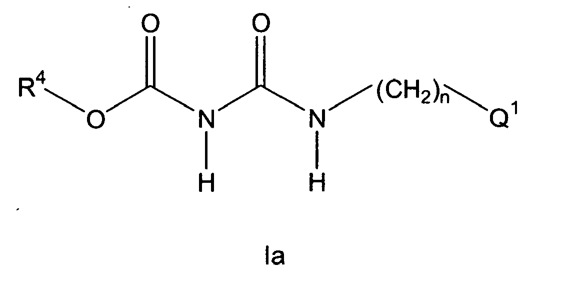 in which
n represents 1, 2 or 3;
Q¹ represents a residue, which is selected from the group consisting of 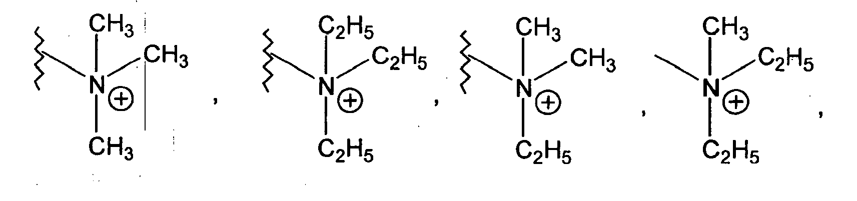 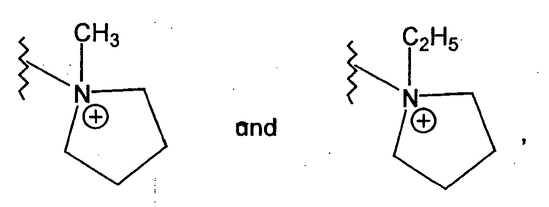 R⁴ represents a linear or branched C₁₋₂₀ alkyl residue;
a phenyl or benzyl residue, and the cyclic moiety of these residues can be unsubstituted or can be substituted with 1, 2, 3, 4 or 5 substituents selected from the group consisting of F, Cl, Br, linear or branched C₁₋₃ alkyl, linear or branched C₁₋₃ alkoxy, phenyl, phenoxy, benzyl and benzyloxy;
or represents a residue selected from the group consisting of 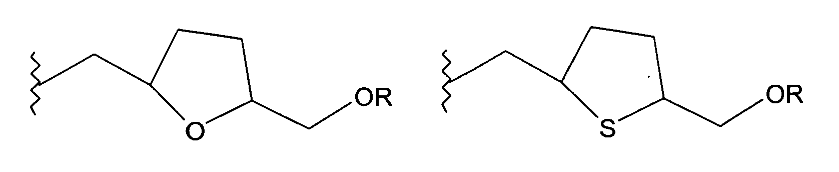 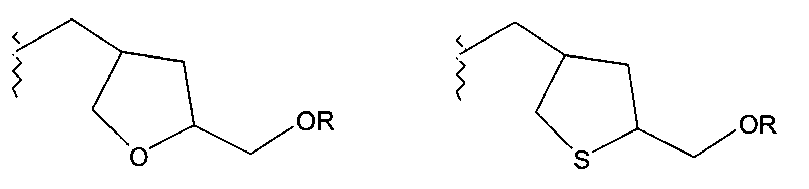 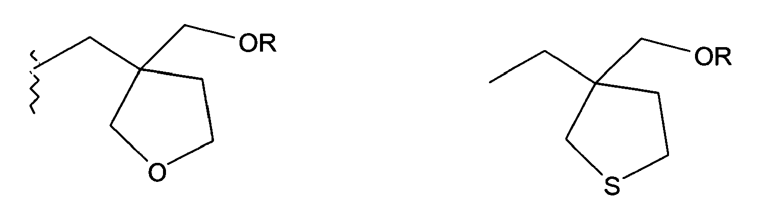 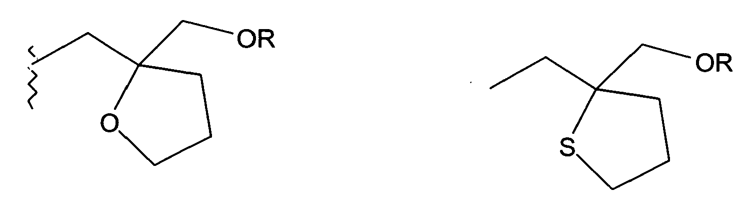 in which R in each case represents a linear or branched C₁₋₂₀-alkyl residue.

6. Salts according to claim 5, **characterised in that**
n represents 1, 2 or 3,
Q¹ represents a residue, which is selected from the group consisting of 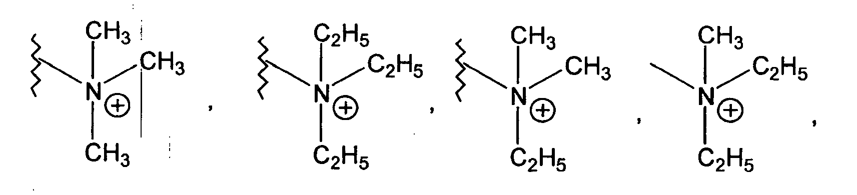 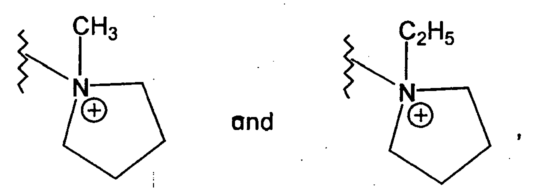 R⁴ represents an alkyl residue selected from the group consisting of methyl, ethyl, n-propyl, n-butyl, n-pentyl, n-hexyl, n-heptyl, n-octyl, n-nonyl, n-decanyl, n-undecanyl, n-dodecanyl, n-tridecanyl, n-tetradecanyl, n-pentadecanyl, n-hexadecanyl, n-heptadecanyl, n-octadecanyl, n-nonadecanyl and n-eicosanyl;
a phenyl or benzyl residue, and the cyclic moiety of these residues can be unsubstituted or substituted once with a substituent selected from the group consisting of F, Cl, Br, linear or branched C₁₋₃ alkyl, linear or branched C₁₋₃ alkoxy, phenyl, phenoxy, benzyl and benzyloxy;
or represents one of the following residues, 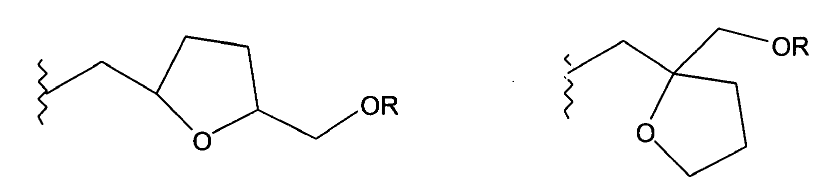 in which R in each case represents an alkyl residue selected from the group consisting of methyl, ethyl, n-propyl, n-butyl, n-pentyl, n-hexyl, n-heptyl, n-octyl, n-nonyl, n-decanyl, n-undecanyl, n-dodecanyl, n-tridecanyl, n-tetradecanyl, n-pentadecanyl, n-hexadecanyl, n-heptadecanyl, n-octadecanyl, n-nonadecanyl and n-eicosanyl.

7. Salts according to one or more of claims 1-6, **characterised in that** the anionic salt partner is a halide ion or a hydroxyl ion, preferably a chloride, bromide or iodide ion, especially preferably an iodide ion.

8. Salts of substituted allophanates according to one or more of claims 1-7, selected from the group consisting of
[1] 4-(Trimethylamino-1-ethyl)allophanic acid dodecyl ester iodide,
[2] 4-(Pyrrolidinium-1-ethyl)allophanic acid hexadecyl ester iodide,
[3] 4-(Trimethylamino-1-propyl)allophanic acid hexadecyl ester iodide,
[4] 4-(Trimethylamino-1-ethyl)allophanic acid octyl ester iodide,
[5] 4-(Trimethylamino-l-ethyl)allophanic acid butyl ester iodide,
[6] 4-(Trimethylamino-1-ethyl)allophanic acid phenyl ester iodide,
[7] 4-Benzyloxycarbonyl-4-(N-methyldimethylammonium-1-ethyl)allophanic acid benzyl ester iodide,
[8] 4-(Trimethylamino-1-ethyl)allophanic acid benzyl ester iodide,
[9] 4-(Trimethylamino-1-ethyl)allophanic acid (4-phenyl)-phenyl ester iodide,
[10] 4-(Trimethylamino-1-ethyl)allophanic acid ethyl ester iodide,
[11] 4-(Trimethylamino-1-ethyl)allophanic acid butyl ester iodide,
[12] 4-(Trimethylamino-1-ethyl)allophanic acid hexyl ester iodide,
[13] 4-(Trimethylamino-1-ethyl)allophanic acid (cis-5-hexadecyloxymethyltetrahydrofuran-2-yl)methyl ester iodide,
[14] 4-(Trimethylamino-1-ethyl)allophanic acid 3-hexadecyloxy-2-methoxypropan-1-yl ester iodide,
[15] 4-(Trimethylamino-1-ethyl)allophanic acid hexadecyl ester iodide and
[16] 4-(Trimethylamino-1-ethyl)allophanic acid (2-hexadecyloxymethyltetrahydrofuran-2-yl)methyl ester iodide.

9. Method for the production of substituted allophanates according to one or more of claims 1-8, **characterised in that**
a compound of general formula II, 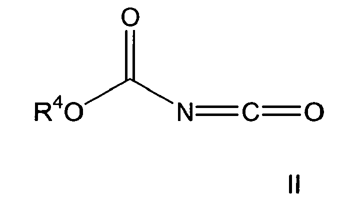 in which R⁴ has the meaning according to one or more of claims 1-8, is reacted in a reaction medium, optionally in the presence of a base, with a compound of general formula III 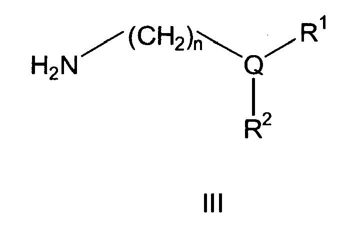 in which n, R¹ and R² have the meaning according to one or more of claims 1-8, to form a compound of general formula IV, 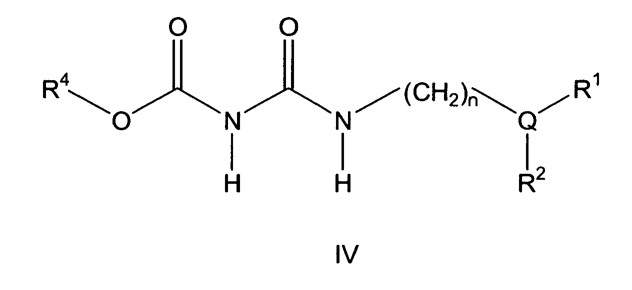 in which R¹, R², R⁴ and n have the meaning stated previously, and the compound of general formula IV thus obtained is converted, by reaction with an alkylating agent, to a compound of general formula I.

10. Medicaments containing at least one salt according to one or more of claims 1 to 8 and optionally one or more physiologically compatible excipients.

11. Medicaments according to claim 10 for the treatment and/or prophylaxis of one or more diseases selected from the group consisting of pain, preferably of pain selected from the group consisting of acute pain, chronic pain, neuropathic pain and visceral pain; arthralgia; migraine; depression; nervous complaints; nerve damage; neurodegenerative diseases, preferably selected from the group consisting of multiple sclerosis, Alzheimer's disease, Parkinson's disease and Huntington's disease; cognitive dysfunctions, preferably cognitive impairment, especially preferably memory disorders; epilepsy; respiratory tract diseases, preferably selected from the group consisting of asthma and pneumonia; cough; urinary incontinence; overactive bladder (OAB); gastric ulcers; irritable bowel syndrome; cerebrovascular accidents; eye irritations; skin irritations; neurotic skin diseases; inflammatory diseases, preferably intestinal inflammations; diarrhoea; pruritus; eating disorders, preferably selected from the group consisting of bulimia, cachexia, anorexia and obesity; dependence on medicaments; abuse of medicaments; withdrawal effects in dependence on medicaments; development of tolerance to medicaments, preferably to natural or synthetic opioids; drug dependence; drug abuse; withdrawal effects in drug dependence; alcohol dependence; alcohol abuse and withdrawal effects in alcohol dependence; for diuresis; for antinatriuresis; for exerting an influence on the cardiovascular system; for increasing alertness; for increasing libido; for modulation of motor activity; for anxiety reduction; for local anaesthesia and/or for suppression of undesirable side effects, preferably selected from the group consisting of hyperthermia, hypertension and bronchial constriction, induced by the administration of vanilloid receptor 1 (VR1/TRPV1 receptors) agonists, preferably selected from the group consisting of capsaicin, resiniferatoxin, olvanil, arvanil, SDZ-249665, SDZ-249482, nuvanil and capsavanil.

12. Use of at least one salt according to one or more of claims 1 to 8 for the production of a medicament for the treatment and/or prophylaxis of one or more diseases selected from the group consisting of pain, preferably of pain selected from the group consisting of acute pain, chronic pain, neuropathic pain and visceral pain; arthralgia; migraine; depression; nervous complaints; nerve damage; neurodegenerative diseases, preferably selected from the group consisting of multiple sclerosis, Alzheimer's disease, Parkinson's disease and Huntington's disease; cognitive dysfunctions, preferably cognitive impairment, especially preferably memory disorders; epilepsy; respiratory tract diseases, preferably selected from the group consisting of asthma and pneumonia; cough; urinary incontinence; overactive bladder (OAB); gastric ulcers; irritable bowel syndrome; cerebrovascular accidents; eye irritations; skin irritations; neurotic skin diseases; inflammatory diseases, preferably intestinal inflammations; diarrhoea; pruritus; eating disorders, preferably selected from the group consisting of bulimia, cachexia, anorexia and obesity; dependence on medicaments; abuse of medicaments; withdrawal effects in dependence on medicaments; development of tolerance to medicaments, preferably to natural or synthetic opioids; drug dependence; drug abuse; withdrawal effects in drug dependence; alcohol dependence; alcohol abuse and withdrawal effects in alcohol dependence; for diuresis; for antinatriuresis; for exerting an influence on the cardiovascular system; for increasing alertness; for increasing libido; for modulation of motor activity; for anxiety reduction; for local anaesthesia and/or for suppression of undesirable side effects, preferably selected from the group consisting of hyperthermia, hypertension and bronchial constriction, induced by the administration of vanilloid receptor 1 (VR1/TRPV1 receptors) agonists, preferably selected from the group consisting of capsaicin, resiniferatoxin, olvanil, arvanil, SDZ-249665, SDZ-249482, nuvanil and capsavanil (DA-5018).

13. Use of at least one salt according to one or more of claims 1 to 8 for the production of a medicament for the treatment and/or prophylaxis of pain selected from the group consisting of acute pain, chronic pain, neuropathic pain and visceral pain.

## Revendications

1. Sels d'esters d'acide allophanique substitués, à base d'un composant cationique du sel de formule générale I, 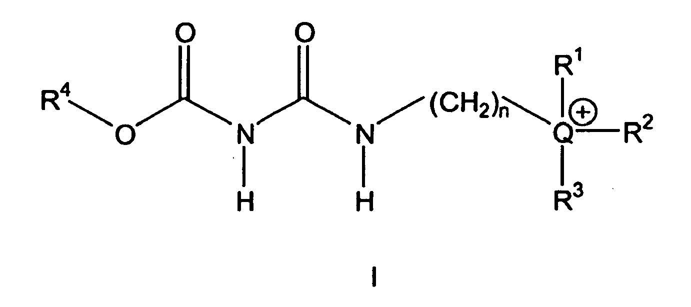 dans laquelle
n = 1, 2, 3, 4, 5 ou 6 ;
Q représente N ou P ;
R¹, R², R³ représentent, chacun indépendamment, un radical alkyle en C₁-C₅ linéaire ou ramifié ;
ou deux de ces radicaux forment ensemble, avec l'atome Q qui les relie en tant que chaînon de cycle, un hétérocycle à 4, 5, 6 ou 7 chaînons, saturé ou insaturé, non substitué ou une ou plusieurs fois substitué, comportant éventuellement au moins un autre hétéroatome en tant que chaînon de cycle, et le radical restant a dans chaque cas la signification donnée précédemment ;
R⁴ représente un radical aliphatique linéaire ou ramifié, saturé ou insaturé, non substitué ou une ou plusieurs fois substitué, comportant éventuellement au moins un hétéroatome en tant que chaînon de la chaîne,
un radical cycloaliphatique saturé ou insaturé, non substitué ou une ou plusieurs fois substitué, comportant éventuellement au moins un hétéroatome en tant que chaînon de cycle, qui peut être lié par un groupe alkylène linéaire ou ramifié,
un radical aryle ou un radical hétéroaryle non substitué ou au moins une fois ou plusieurs fois substitué, éventuellement lié par un groupe alkylène linéaire ou ramifié, ou
représente
un groupe -(CH₂)_{q}-Aᵣ-(CH₂)ₛ-B-(CH₂)ₜ-Cᵤ-R⁵ dans lequel
q, s et t représentent chacun indépendamment 0, 1, 2, 3, 4, 5 ou 6 ;
r et u représentent, indépendamment l'un de l'autre, 0 ou 1 ;
A et C représentent, indépendamment l'un de l'autre, 0, S ou NH ;
B représente un groupe alkylène, alcénylène ou alcynylène linéaire ou ramifié, non substitué ou une ou plusieurs fois substitué ; un radical cycloaliphatique saturé ou insaturé, non substitué ou une ou plusieurs fois substitué, comportant éventuellement au moins un hétéroatome en tant que chaînon de cycle, ou un radical aryle ou hétéroaryle non substitué ou une ou plusieurs fois substitué ; et
R⁵ représente un radical aliphatique linéaire ou ramifié, saturé ou insaturé, non substitué ou une ou plusieurs fois substitué,
chaque fois éventuellement sous forme d'un de ses stéréoisomères purs, en particulier énantiomères ou diastéréoisomères, de son racémate ou sous forme d'un mélange des stéréoisomères, en particulier des énantiomères et/ou diastéréoisomères, en un rapport quelconque de mélange,
et d'un composant anionique du sel.

2. Sels selon la revendication 1, **caractérisés en ce que**
R¹, R², R³ représentent, chacun indépendamment, un radical alkyle qui est choisi dans l'ensemble constitué par les groupes méthyle, éthyle, n-propyle et isopropyle,
ou deux de ces radicaux forment ensemble, avec l'atome Q qui les relie en tant que chaînon de cycle, un hétérocycle à 4, 5, 6 ou 7 chaînons, saturé ou insaturé, non substitué ou une ou plusieurs fois substitué, comportant éventuellement au moins un autre hétéroatome en tant que chaînon de cycle, et le radical restant dans chaque cas représente un radical alkyle qui est choisi dans l'ensemble constitué par les groupes méthyle, éthyle, n-propyle et isopropyle.

3. Sels selon la revendication 1 ou 2, **caractérisés en ce que**
R⁴ représente un radical aliphatique en C₁-C₃₀ linéaire ou ramifié, saturé ou insaturé, non substitué ou une ou plusieurs fois substitué ;
un radical cycloaliphatique à 5, 6 ou 7 chaînons, saturé ou insaturé, non substitué ou une ou plusieurs fois substitué, comportant éventuellement un, deux ou trois hétéroatomes en tant que chaînon de cycle, qui peut être lié par un groupe alkylène en C₁-C₃ linéaire ou ramifié,
un radical aryle ou un radical hétéroaryle à 5 ou 6 chaînons, non substitué ou au moins monosubstitué, éventuellement lié par un groupe alkylène en C₁-C₃ linéaire ou ramifié, ou
représente
un groupe -(CH₂)_{q}-Aᵣ-(CH₂)ₛ-B-(CH₂)ₜ-Cᵤ-R⁵ dans lequel
q, s et t représentent chacun indépendamment 0, 1, 2, 3, 4, 5 ou 6 ;
r et u représentent, indépendamment l'un de l'autre, 0 ou 1 ;
A et C représentent, indépendamment l'un de l'autre, O ou S ;
B représente un groupe alkylène en C₁-C₆, alcénylène en C₂-C₆ ou alcynylène en C₂-C₆ linéaire ou ramifié, qui peut porter 1, 2 ou 3 substituants choisis chacun indépendamment dans l'ensemble constitué par F, Cl, Br, OH, -O-alkyle(C₁-C₃), SH et -S-alkyle(C₁-C₃),
représente un radical (hétéro)cycloaliphatique choisi dans l'ensemble constitué par les groupes cyclopropyle, cyclobutyle, cyclopentyle, cyclohexyle, cyclohexyle, cyclo-octyle, cyclononyle, cyclopentényle, cyclohexényle, cycloheptényle, cyclo-octényle, imidazolinyle, aziridinyle, azétidinyle, pyrrolidinyle, pipéridinyle, azépanyle, azokanyle, pipérazinyle, tétrahydrofurannyle (tétrahydrofuryle), tétrahydrothiényle (tétrahydrothiophényle), morpholinyle et thiomorpholinyle,
ou représente un radical (hétéro)aryle choisi dans l'ensemble constitué par les groupes pyrrolyle, indolyle, furyle (furannyle), benzo[b]furannyle, thiényle (thiophényle), benzo[b]thiényle, pyrazolyle, imidazolyle, thiazolyle, thiadiazolyle, triazolyle, oxazolyle, isoxazolyle, pyridinyle, pyridazinyle, pyrimidinyle, pyrazinyle, pyrannyle, indazolyle, purinyle, indolizinyle, quinolinyle, isoquinolinyle et quinazolinyle, et
R⁵ représente un radical aliphatique en C₁-C₃₀ linéaire ou ramifié, saturé ou insaturé, non substitué ou une ou plusieurs fois substitué,

4. Sels selon une ou plusieurs des revendications 1 à 3, **caractérisés en ce que**
n = 1, 2, 3, 4, 5 ou 6 ;
Q représente N ou P ;
R¹, R², R³ représentent, chacun indépendamment, un radical alkyle qui est choisi dans l'ensemble constitué par les groupes méthyle, éthyle, n-propyle et isopropyle,
ou deux de ces radicaux forment ensemble, avec l'atome Q qui les relie en tant que chaînon de cycle, un hétérocycle à 4, 5, 6 ou 7 chaînons, saturé, non substitué, et le radical restant dans chaque cas représente un radical alkyle qui est choisi dans l'ensemble constitué par les groupes méthyle, éthyle, n-propyle et isopropyle,
R⁴ représente un radical aliphatique en C₁-C₂₀ linéaire ou ramifié, saturé ou insaturé ;
représente un radical aryle ou hétéroaryle éventuellement lié par un groupe alkylène en C₁-C₃, choisi dans l'ensemble constitué par les groupes phényle, naphtyle, furannyle, thiophényle et pyridinyle, le fragment cyclique de ces radicaux dans chaque cas étant non substitué ou pouvant porter 1, 2, 3, 4 ou 5 substituants choisis dans le groupe constitué par F, Cl, Br, un groupe alkyle en C₁-C₃ linéaire ou ramifié, alcoxy en C₁-C₃ linéaire - ou ramifié, phényle, phénoxy, benzyle ou benzyloxy ;
représente
un groupe -(CH₂)_{q}-Aᵣ-(CH₂)ₛ-B-(CH₂)ₜ-Cᵤ-R⁵ dans lequel
q, s et t représentent chacun indépendamment 0, 1 ou 2 ;
r et u représentent chacun, indépendamment l'un de l'autre, 0 ou 1 ;
A et C représentent, indépendamment l'un de l'autre, un atome d'oxygène ;
B représente un groupe alkylène en C₁-C₆, alcénylène en C₂-C₆ ou alcynylène en C₂-C₆ linéaire ou ramifié, qui peut porter 1, 2 ou 3 substituants choisis chacun indépendamment dans l'ensemble constitué par F, Cl, Br, OH, -O-alkyle(C₁-C₃), SH et -S-alkyle(C₁-C₃),
représente un radical (hétéro)-cycloaliphatique choisi dans l'ensemble constitué par les groupes cyclopropyle, cyclobutyle, cyclopentyle, cyclohexyle, cyclohexyle, cyclo-octyle, cyclononyle, cyclopentényle, cyclohexényle, cycloheptényle, cyclo-octényle, imidazolinyle, aziridinyle, azétidinyle, pyrrolidinyle, pipéridinyle, azépanyle, azokanyle, pipérazinyle, tétrahydrofurannyle (tétrahydrofuryle), tétrahydrothiényle (tétrahydrothiophényle), morpholinyle et thiomorpholinyle,
ou représente un radical (hétéro)aryle choisi dans l'ensemble constitué par les groupes pyrrolyle, indolyle, furyle (furannyle), benzo[b]furannyle, thiényle (thiophényle), benzo[b]thiényle, pyrazolyle, imidazolyle, thiazolyle, thiadiazolyle, triazolyle, oxazolyle, isoxazolyle, pyridinyle, pyridazinyle, pyrimidinyle, pyrazinyle, pyrannyle, indazolyle, purinyle, indolizinyle, quinolinyle, isoquinolinyle et quinazolinyle, et
R⁵ représente un radical aliphatique en C₁-C₂₀ linéaire ou ramifié, saturé ou insaturé.

5. Sels selon une ou plusieurs des revendications 1 à 4, à base d'un composant cationique du sel de formule générale la 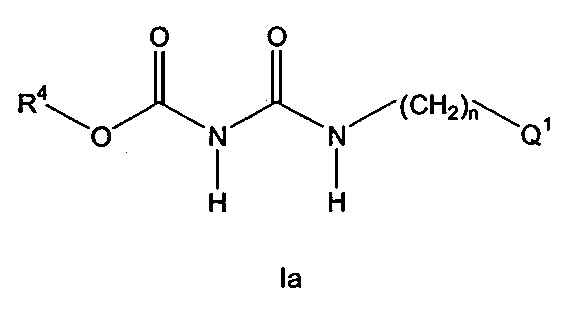 dans laquelle
n = 1, 2 ou 3 ;
Q¹ représente un radical qui est choisi dans l'ensemble constitué par 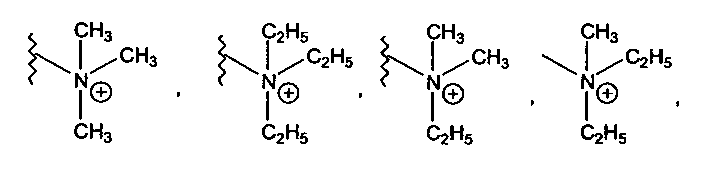 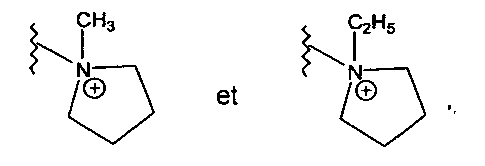
R⁴ représente un radical alkyle en C₁-C₂₀ ;
un radical phényle ou benzyle, le fragment cyclique de ces radicaux dans chaque cas étant non substitué ou pouvant porter 1, 2, 3, 4 ou 5 substituants choisis dans l'ensemble constitué par F, Cl, Br, un groupe alkyle en C₁-C₃ linéaire ou ramifié, alcoxy en C₁-C₃ linéaire ou ramifié, phényle, phénoxy, benzyle ou benzyloxy ;
ou représente un radical choisi dans l'ensemble constitué par 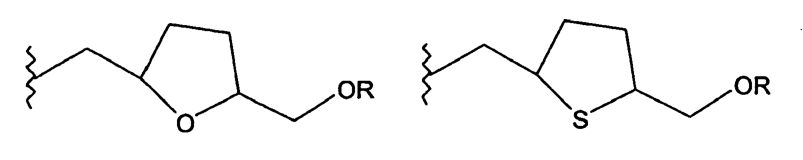 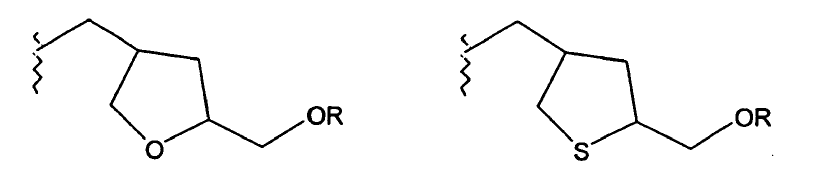 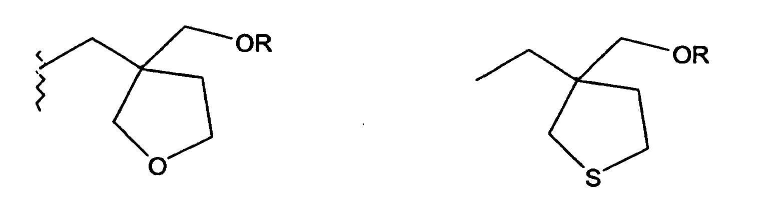 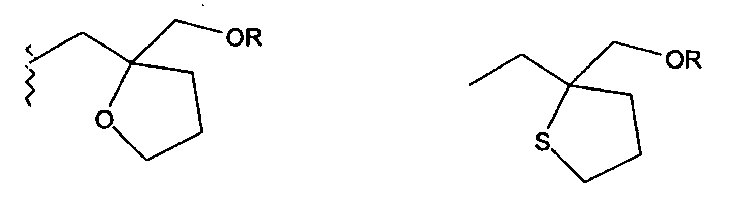 où R représente chaque fois un radical alkyle en C₁-C₂₀ linéaire ou ramifié.

6. Sels selon la revendication 5, **caractérisée en ce que**
n = 1, 2 ou 3 ;
Q¹ représente un radical qui est choisi dans l'ensemble constitué par 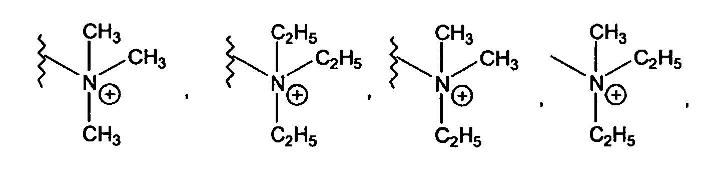 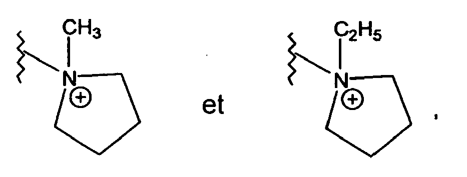
R⁴ représente un radical alkyle choisi dans l'ensemble constitué par les groupes méthyle, éthyle, n-propyle, n-butyle, n-pentyle, n-hexyle, n-heptyle, n-octyle, n-nonyle, n-décanyle, n-undécanyle, n-dodécanyle, n-tridécanyle, n-tétradécanyle, n-pentadécanyle, n-hexadécanyle, n-heptadécanyle, n-octadécanyle, n-nonadécanyle et n-eicosanyle ;
un radical phényle ou benzyle, le fragment cyclique de ces radicaux étant non substitué ou pouvant porter un substituant choisi dans l'ensemble constitué par F, Cl, Br, un groupe alkyle en C₁-C₃ linéaire ou ramifié, alcoxy en C₁-C₃ linéaire ou ramifié, phényle, phénoxy, benzyle ou benzyloxy ;
ou représente l'un des radicaux suivants, 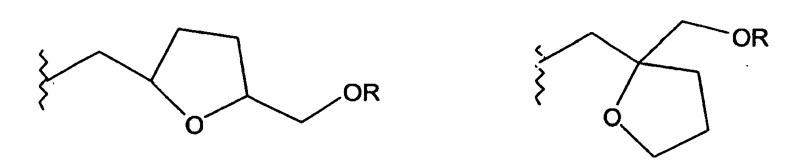 où R représente chaque fois un radical alkyle choisi dans l'ensemble constitué par les groupes méthyle, éthyle, n-propyle, n-butyle, n-pentyle, n-hexyle, n-heptyle, n-octyle, n-nonyle, n-décanyle, n-undécanyle, n-dodécanyle, n-tridécanyle, n-tétradécanyle, n-pentadécanyle, n-hexadécanyle, n-heptadécanyle, n-octadécanyle, n-nonadécanyle et n-eicosanyle.

7. Sels selon une ou plusieurs des revendications 1 à 6, **caractérisés en ce que** le composant anionique du sel est un ion halogénure ou un ion hydroxy, de préférence un ion chlorure, bromure ou iodure, en particulier un ion iodure.

8. Sels d'esters d'acide allophanique substitués, selon une ou plusieurs des revendications 1 à 7, choisis dans l'ensemble constitué par
[1] l'iodure de 4-(triméthylamino-1-éthyl)-allophanate de dodécyle,
[2] l'iodure de 4-(pyrrolidinium-1-éthyl)-allophanate d'hexadécyle,
[3] l'iodure de 4-(triméthylamino-1-propyl)-allophanate d'hexadécyle,
[4] l'iodure de 4-(triméthylamino-1-éthyl)-allophanate d'octyle,
[5] l'iodure de 4-(triméthylamino-1-éthyl)-allophanate de butyle,
[6] l'iodure de 4-(triméthylamino-1-éthyl)-allophanate de phényle,
[7] l'iodure de 4-benzyloxycarbonyl-4-(N-méthyldiméthylammonium-1-éthyl)allophanate de benzyle,
[8] l'iodure de 4-(triméthylamino-1-éthyl)-allophanate de benzyle,
[9] l'iodure de 4-(triméthylamino-1-éthyl)-allophanate de (4-phényl)-phényle,
[10] l'iodure de 4-(triméthylamino-1-éthyl)-allophanate d'éthyle,
[11] l'iodure de 4-(triméthylamino-1-éthyl)-allophanate de butyle,
[12] l'iodure de 4-(triméthylamino-1-éthyl)-allophanate d'hexyle,
[13] l'iodure de 4-(triméthylamino-1-éthyl)-allophanate de (*cis*-5-hexadécyloxyméthyltétrahydrofurann-2-yl)méthyle,
[14] l'iodure de 4-(triméthylamino-1-éthyl)-allophanate de 3-hexadécyloxy-2-méthoxypropan-1-yle,
[15] l'iodure de 4-(triméthylamino-1-éthyl)-allophanate d'hexadécyle et
[16] l'iodure de 4-(triméthylamino-1-éthyl)-allophanate de (2-hexadécyloxyméthyltétrahydrofurann-2-yl)méthyle.

9. Procédé pour la préparation d'esters d'acide allophanique substitués selon une ou plusieurs des revendications 1 à 8, **caractérisée en ce qu'**on fait réagir
un composé de formule générale II, 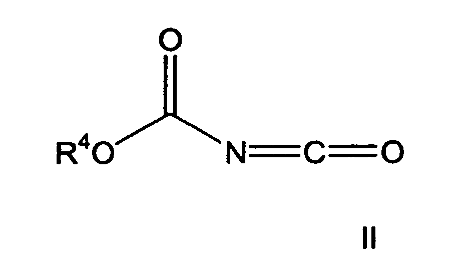 dans laquelle R⁴ a la signification selon une ou plusieurs des revendications 1 à 8, dans un milieu réactionnel, éventuellement en présence d'une base, avec un composé de formule générale III 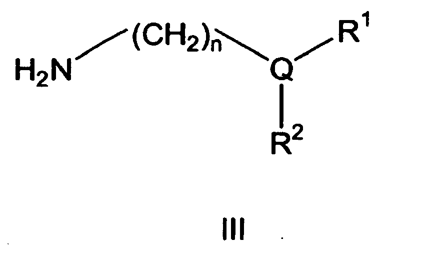 dans laquelle n, R¹ et R² ont les significations selon une ou plusieurs des revendications 1 à 8, pour obtenir un composé de formule générale IV 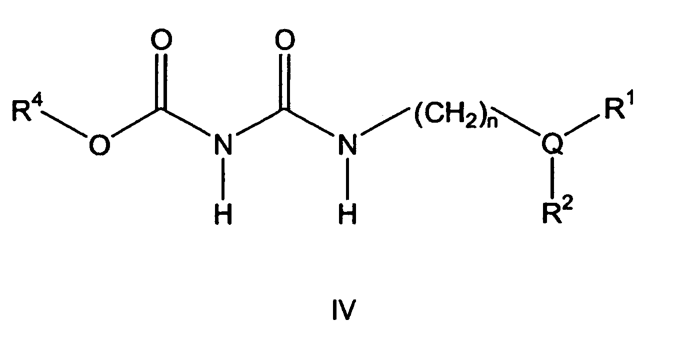 dans laquelle R¹, R², R⁴ et n ont les significations données précédemment, et on convertit le composé de formule générale IV ainsi obtenu en un composé de formule générale I en le faisant réagir avec un agent d'alkylation.

10. Médicament contenant au moins un sel selon une ou plusieurs des revendications 1 à 8 et éventuellement un ou plusieurs adjuvants physiologiquement acceptables.

11. Médicament selon la revendication 10, destiné au traitement et/ou à la prophylaxie d'une ou plusieurs affections choisies dans le groupe constitué par la douleur, de préférence la douleur choisie dans l'ensemble consistant en la douleur aiguë, la douleur chronique, la douleur névropathique et la douleur viscérale ; l'arthralgie ; la migraine ; les dépressions ; les neuropathies ; les lésions nerveuses ; les maladies neurodégénératives, choisies de préférence dans l'ensemble constitué par la sclérose en plaques, la maladie d'Alzheimer, la maladie de Parkinson et la chorée de Huntington ; les dysfonctionnements cognitifs, de préférence les états de déficience cognitive, de façon particulièrement préférée les troubles de la mémoire ; l'épilepsie ; les affections des voies respiratoires, choisies de préférence dans le groupe consistant en l'asthme et l'inflammation pulmonaire ; la toux ; l'incontinence urinaire ; une vessie hyperactive (*overactive bladder,* OAB) ; les ulcères gastriques ; le syndrome de l'intestin irritable ; les accidents vasculaires cérébraux ; les irritations des yeux ; les irritations de la peau ; les maladies névrotiques de la peau ; les maladies inflammatoires, de préférence les inflammations de l'intestin ; la diarrhée ; le prurit ; les troubles de la prise d'aliments, choisis de préférence dans le groupe constitué par la boulimie, la cachexie, l'anorexie, et l'obésité ; la pharmacodépendance ; le mésusage de médicaments ; les manifestations de sevrage dans la pharmacodépendance ; l'échappement thérapeutique vis-à-vis de médicaments, de préférence vis-à-vis d'opioïdes naturels ou synthétiques ; la dépendance aux drogues ; l'usage de drogues ; les manifestations de sevrage dans la dépendance aux drogues ; la dépendance à l'alcool ; l'abus d'alcool et les manifestations de sevrage dans la dépendance alcoolique ; à la diurèse ; à 'anti-natriurie ; à influer sur le système cardiovasculaire ; à l'accroissement de la vigilance ; à la stimulation de la libido ; à la modulation de l'activité motrice ; à l'anxiolyse ; à l'anesthésie locale et/ou à l'inhibition d'effets secondaires indésirables, choisis de préférence dans le groupe consistant en l'hyperthermie, l'hypertension artérielle et le resserrement bronchique, déclenchés par l'administration d'agonistes des récepteurs de vanilloïde 1 (récepteurs VR1/TRPV1), choisis de préférence dans le groupe consistant en la capsaïcine, la résinifératoxine, l'olvanil, l'arvanil, SDZ-249665, SDZ-249482, le nuvanil et le capsavanil.

12. Utilisation d'au moins un sel selon une ou plusieurs des revendications 1 à 8, pour la fabrication d'un médicament destiné au traitement et/ou à la prophylaxie d'une ou plusieurs affections choisies dans le groupe constitué par la douleur, de préférence la douleur choisie dans l'ensemble consistant en la douleur aiguë, la douleur chronique, la douleur névropathique et la douleur viscérale ; l'arthralgie ; la migraine ; les dépressions ; les neuropathies ; les lésions nerveuses ; les maladies neurodégénératives, choisies de préférence dans l'ensemble constitué par la sclérose en plaques, la maladie d'Alzheimer, la maladie de Parkinson et la chorée de Huntington ; les dysfonctionnements cognitifs, de préférence les états de déficience cognitive, de façon particulièrement préférée les troubles de la mémoire ; l'épilepsie; les affections des voies respiratoires, choisies de préférence dans le groupe consistant en l'asthme et l'inflammation pulmonaire ; la toux ; l'incontinence urinaire ; une vessie hyperactive (*overactive bladder,* OAB) ; les ulcères gastriques ; le syndrome de l'intestin irritable ; les accidents vasculaires cérébraux ; les irritations des yeux ; les irritations de la peau ; les maladies névrotiques de la peau ; les maladies inflammatoires, de préférence les inflammations de l'intestin ; la diarrhée ; le prurit ; les troubles de la prise d'aliments, choisis de préférence dans le groupe constitué par la boulimie, la cachexie, l'anorexie, et l'obésité ; la pharmacodépendance ; le mésusage de médicaments ; les manifestations de sevrage dans la pharmacodépendance ; l'échappement thérapeutique vis-à-vis de médicaments, de préférence vis-à-vis d'opioïdes naturels ou synthétiques ; la dépendance aux drogues ; l'usage de drogues ; les manifestations de sevrage dans la dépendance aux drogues ; la dépendance à l'alcool ; l'abus d'alcool et les manifestations de sevrage dans la dépendance alcoolique ; à la diurèse ; à 'anti-natriurie ; à influer sur le système cardiovasculaire ; à l'accroissement de la vigilance ; à la stimulation de la libido ; à la modulation de l'activité motrice ; à l'anxiolyse ; à l'anesthésie locale et/ou à l'inhibition d'effets secondaires indésirables, choisis de préférence dans le groupe consistant en l'hyperthermie, l'hypertension artérielle et le resserrement bronchique, déclenchés par l'administration d'agonistes des récepteurs de vanilloïde 1 (récepteurs VR1/TRPV1), choisis de préférence dans le groupe consistant en la capsaïcine, la résinifératoxine, l'olvanil, l'arvanil, SDZ-249665, SDZ-249482, le nuvanil et le capsavanil (DA-5018).

13. Utilisation d'au moins un sel selon une ou plusieurs des revendications 1 à 8, pour la fabrication d'un médicament destiné au traitement et/ou à la prophylaxie de la douleur choisie dans le groupe consistant en la douleur aiguë, la douleur chronique, la douleur névropathique et la douleur viscérale.
